# EUROPEAN PATENT APPLICATION

(11) **EP 4 613 290 A1**
(43) Date of publication of application: **10.09.2025**
(21) Application number: 23885056.4
(22) Date of filing: 02.11.2023
(51) Int. Cl.: A61K 47/68, A61K 35/17, C07D 495/04, A61P 35/00

(54) **TARGETING MOLECULE-CELL COMPLEX AND PREPARATION METHOD THEREFOR**

(30) Priority: 02.11.2022 US 202263421684 P
(71) Applicant: TCI GENE INC, Taipei City 114 (TW)
(72) Inventor: LIN, Yung-Hsiang, Taipei City Taiwan (TW); GU, Ming-Shian, Taipei City Taiwan (TW)
(74) Representative: Michalski Hüttermann & Partner Patentanwälte mbB
(86) International application number: PCT/CN2023/129372
(87) International publication number: WO 2024/094131

(57) **Abstract**

Provided are a targeting molecule-cell complex capable of being used for the treatment and/or prevention of cancer, and a preparation method therefor. The targeting molecule-cell complex is a complex having formula (I) [X-A₁-L-A₂-D], wherein: X is a cell; A1 is a substituted or unsubstituted indazolone moiety; L is -O-(CH₂)ₘ-W-(CH₂)ₙ-, wherein m and n are each independently an integer between 0 and 10, and W is a single bond or -NHCO- or a substituted or unsubstituted polyethylene glycolene (PEG) with 1-4 units; A₂ is -CONH- or -COS -; and D is a targeting moiety.

## Description

### Technical Field

The present invention relates to the treatment and/or prevention of cancer, and in particular, it relates to a targeting molecule-cell complex comprising a specific linker and a preparation method thereof.

### Background Art

In recent years, cell therapy technologies have made significant progress in the field of cancer treatment. Among these, although conventional cell therapies involving the injection of autologous or allogeneic natural killer cells (NK cell) or T cells into cancer patients can demonstrate a certain degree of efficacy in the human body, the activity and effectiveness of such cells are limited by the molecules expressed on their surfaces. Taking conventional non-genetically modified T cells as an example, these T cells are only limited to responding to tumor antigen peptides presented by the major histocompatibility complex (MHC), but cancer cells have low expression of the major histocompatibility complex, which limits the therapeutic effect.

In this regard, existing technologies have employed synthetic chimeric antigen receptors (CAR) to bypass the limitations imposed by major histocompatibility complex, thereby directly exerting specific cytotoxicity on target molecules on the surface of malignant cells. Among such technologies, chimeric antigen receptor T cell therapies (CAR-T cell therapy) which are used for the treatment of acute lymphoblastic leukemia and non-Hodgkin lymphoma (such as Kymriah^{®} and Yescarta^{®}), have successfully demonstrated the potential of immune cells in the advancement of cancer immunotherapy. However, the manufacturing process of CAR-T cells remains technically challenging, costly, and time-consuming, and raises safety concerns such as the potential for viral vector residues.

In summary, existing technologies in the treatment or prevention of cancer are not entirely satisfactory in all respects, and thus further improvements are required.

### Summary of the invention

The invention is defined by the features of the independent claims.

In view of the above, the present invention provides a crosslinked complex molecule and a crosslinking method, which includes a photo-reactive functional group moiety and a targeting molecule moiety, and the photo-reactive functional group moiety can bind to an amino functional group (-NH₂) on cell surface proteins by irradiating ultraviolet (UV) light. The crosslinked complex molecule provided by the present invention can also, in the condition of conjugation with a targeting molecule, undergo ultraviolet light irradiation to bind to the amino functional group on the cell surface proteins, thereby making the cell carry the targeting molecule to enhance its efficacy in disease treatment and prevention. Thus, the present invention provides a faster and less costly cell therapeutic drugs and methods of manufacturing them.

Based on the above, the present invention primarily provides a targeting molecule-cell complex for the treatment and/or prevention of cancer, comprising any one of the following configurations [1]-[13]:
[1] A targeting molecule-cell complex having the formula (I):

   X-A₁-L-A₂-D Formula (I)

   in which:
   X is a cell;
   A₁ is a substituted or unsubstituted indazolone moiety;
   L is -O-(CH₂)ₘ-W-(CH₂)ₙ-, in which m and n are each independently integers from 0 to 10, and W is a single bond, or -NHCO-, or a substituted or unsubstituted polyethylene glycol (PEG) with 1-4 units;
   A₂ is -CONH- or -COS-; and
   D is a targeting moiety.
[2] The targeting molecule-cell complex as described in [1], in which A₁ is a methoxy-substituted indazolone group.
[3] The targeting molecule-cell complex as described in [2], having a structure of Formula (II) or (III), 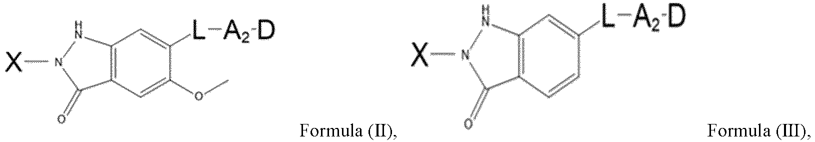 in which X, L, A₂, and D are as defined in [1].
[4] The targeting molecule-cell complex as described in [1], in which L is -O-(CH₂)ₘ₊ₙ-, and in which 3≤(m+n)≤9.
[5] The targeting molecule-cell complex as described in [1], in which L is -O-(CH₂)ₘ-NHCO-(CH₂)ₙ-, and 3≤(m+n)≤9.
[6] The targeting molecule-cell complex as described in [1], in which L is the substituted or unsubstituted polyethylene glycol with 1-4 units.
[7] The targeting molecule-cell complex as described in [1], in which the cell is a mesenchymal stem cell, a blood cell, or a bacterial cell.
[8] The targeting molecule-cell complex as described in [7], in which the mesenchymal stem cell comprises adipose-derived mesenchymal stem cell (ADMSC), hematopoietic stem cell, bone marrow mesenchymal stem cell, umbilical cord mesenchymal stem cell, and embryonic stem cell.
[9] The targeting molecule-cell complex as described in [7], in which the blood cell comprises a platelet, a T lymphocyte, a natural killer cell (NK cell), a super natural killer cell (Snk cell), a Cytokine-induced killer cell (CIK cell), a dendritic cell, a macrophage, a granule, or a combination thereof.
[10] The targeting molecule-cell complex as described in [1], in which the targeting moiety is an antigen-binding molecule.
[11] The targeting molecule-cell complex as described in [10], in which the targeting moiety comprises an amino group (NH₂ group) or a sulfhydryl group (sulfhydryl group).
[12] The targeting molecule-cell complex as described in [10], in which the targeting moiety is a small molecule, an aptamer (aptamer), a peptide, an antibody, or a combination thereof.
[13] The targeting molecule-cell complex as described in [11], in which the antibody is a single-chain variable fragment (scFv), a fragment antigen-binding (Fab) fragment, or a full-length antibody.

In addition, the present invention also provides a method of preparing a targeting molecule-cell complex, comprising any one of the following configurations [14] to [23]:
[14] A method of preparing a targeting molecule-cell complex, comprising:
   providing a linker having Formula (IV): 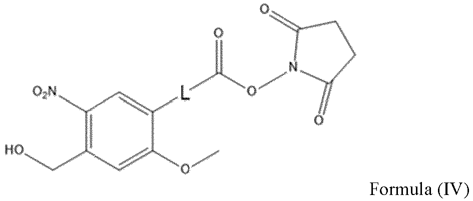
   in which L is -O-(CH₂)ₘ-W-(CH₂)ₙ-, in which m and n are each independently integers from 0 to 10, and W is a single bond, or -NHCO-, or a substituted or unsubstituted polyethylene glycol with 1-4 units;
   reacting the linker with a targeting molecule containing an amino group to form a linker-targeting molecule complex, in which the linker is bonded to the targeting molecule via an amide bond; and
   irradiating the linker-targeting molecule complex with ultraviolet light, such that the irradiated linker-target molecule complex reacts with a cell, in which the linker-targeting molecule complex reacts with the cell through an indazolone moiety to form a targeting molecule-cell complex.
[15] The method of preparing a targeting molecule-cell complex as described in [14], in which the linker has a structure selected from the group consisting of the following compounds: 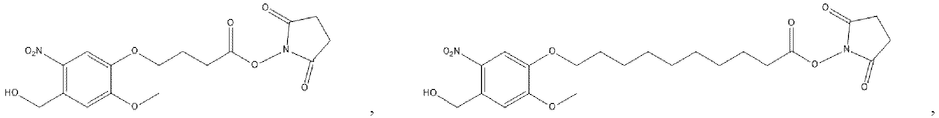 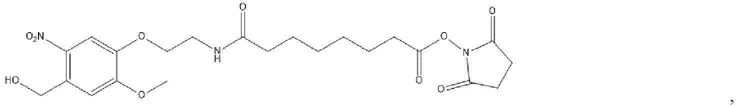 and 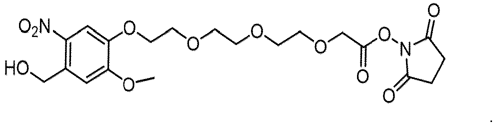
[16] The method of preparing a targeting molecule-cell complex as described in [14], in which a molar ratio of the targeting molecule to the linker used for forming the linker-targeting molecule complex is 1:1 to 1:15.
[17] The method of preparing a targeting molecule-cell complex as described in [14], in which the step of irradiating the linker-targeting molecule complex with ultraviolet light is carried out by irradiating the linker-target molecule complex for 5 to 30 seconds, followed by a standing period of 1 to 30 seconds in a cycle.
[18] The method of preparing a targeting molecule-cell complex as described in [14], in which the cycle is repeated 1 to 6 times.
[19] The method of preparing a targeting molecule-cell complex as described in [14], in which the step of reacting the irradiated linker-target molecule complex with the cell is carried out for at least 1 minute.
[20] The method of preparing a targeting molecule-cell complex as described in [14], in which the step of irradiating the linker-target molecule complex with ultraviolet light comprises first mixing the linker-target molecule complex with the cell, and then irradiating the linker-target molecule complex and the cell with ultraviolet light simultaneously.
[21] Use of the targeting molecule-cell complex as described in [1] in the preparation of a medicament for treating cancer.
[22] Use of the targeting molecule-cell complex of [as described in [1] in the preparation of a medicament for promoting cell homing.
[23] Use of the targeting molecule-cell complex as described in [1] in the preparation of a medicament for treating autoimmune diseases.

Through the configurations described above, the crosslinked complex molecule of the present invention enables the preparation of a targeting molecule-cell complex via a non-genetic modification approach, by a photo-reactive functional group moiety and a targeting molecule conjugating moiety. Accordingly, the crosslinked complex molecule of the present invention has at least the following advantages: (1) it belongs to small molecules and thus is cheap to produce; (2) the photo-reactive functional group bind rapidly to amino functional groups (-NH₂) on cells (with a reaction time of about 1 to 10 minutes) enabling a fast and simple production process; (3) no viral vectors that can insert into human genes are used, thus reducing quality control costs and safety concerns caused by residual viruses in the process; (4) it can broadly bind to amino groups (-NH₂) on different cells; (5) it enhances cellular functionality through the binding of the crosslinked complex molecule-targeting molecule to the cell, for example, enhancing cytotoxicity and inhibiting tumor growth; (6) compared to other linkers, the crosslinking molecule of the present invention exhibits superior cell-conjugating ability and cellular efficacy. In summary, by utilizing the crosslinked complex molecule of the present invention, a targeting molecule-cell complex that can be effectively applied in the treatment and/or prevention of a cancer and preparation methods of the targeting molecule-cell complex can be provided.

### DESCRIPTION OF THE DRAWINGS

Figure 1 shows a preparation flowchart of linker formula α.
Figure 2 shows a nuclear magnetic resonance (NMR) analysis spectrum of linker formula α.
Figure 3 shows a liquid chromatography-tandem mass spectrometry (LC-MS/MS) analysis spectrum of linker formula α.
Figure 4 shows a preparation flowchart of linker formula β (linker formula β).
Figure 5 shows an NMR analysis spectrum of linker formula β.
Figure 6 shows an LC-MS/MS analysis spectrum of linker formula β.
Figure 7 shows a preparation flowchart of linker formula γ.
Figure 8 shows an NMR analysis spectrum of linker formula γ.
Figure 9 shows an LC-MS/MS analysis spectrum of linker formula γ.
Figure 10 shows a preparation flowchart of linker formula δ.
Figure 11 shows an NMR analysis spectrum of linker formula δ.
Figure 12 shows an LC-MS/MS analysis spectrum of linker formula δ.
Figure 13 shows a diagram of the result of sodium dodecyl-sulfate polyacrylamide gel electrophoresis (SDS-PAGE) of different hydrocarbon chain linker conjugated Trastuzumab.
Figure 14 is a diagram that shows a comparative test result of the conjugating degree of different hydrocarbon chain linker conjugated Trastuzumab to ADMSC.
Figure 15 is a diagram that shows a comparative test result of the conjugating degree of different hydrocarbon chain linker conjugated Trastuzumabto NK cells.
Figure 16 is a diagram that shows the effect of different hydrocarbon chain linker conjugated Trastuzumab-NK cells on the cytotoxicity against solid tumor breast cancer cells.
Figure 17 is a diagram that shows the results of the conjugating degree between NK cells and different hydrocarbon chain linker conjugated Trastuzumab.
Figures 18A-18C is a diagram that show the cancer cell apoptosis results of three types of solid tumor cancer cells caused by different hydrocarbon chain linker conjugated Trastuzumab NK cells.
Figure 19 is a diagram that shows the results of linker conjugated Trastuzumab to NK cells at different conjugating reaction times.
Figure 20 is a diagram that shows the conjugating degree of linker conjugated Trastuzumab to the surface of NK cells at different times.
Figure 21 is a diagram that shows the results of the conjugating response of linker formula α conjugated Trastuzumab to platelets.
Figure 22 is a diagram that shows the conjugating ability of linker formula α conjugated Trastuzumab to CD41⁺ platelets.
Figure 23 is a diagram that shows the results of the conjugating response of linker formula α conjugated Trastuzumab to CD3⁺/CD8⁺ T cells.
Figure 24 is a diagram that shows the conjugating ability of linker formula α conjugated Trastuzumab to CD3⁺/CD8⁺ T cells.
Figure 25 is a diagram that shows the conjugating degree of linker conjugated Trastuzumab-CD16+ NK cell membrane at different times.
Figure 26 is a diagram that shows the result of the cell viability after 96 hours of conjugating linker conjugated Trastuzumab to CD16+ NK cells.
Figure 27 is a diagram that shows the results the conjugation between linker conjugated Rituximab and NK cells.
Figure 28 is a diagram that shows the conjugating ability of linker conjugated Rituximab to NK cells.
Figure 29 is a diagram that shows the conjugating results of linker conjugated Atezolizumab to NK cells.
Figure 30 is a diagram that shows the conjugating ability of linker conjugated Atezolizumab to NK cells.
Figure 31 is a diagram that shows the cytotoxicity test results of Rituximab conjugated-PBMC cells.
Figure 32 is a diagram that shows the tumor volume measurement results for BT474 tumors.
Figure 33 is a diagram that shows the tumor growth inhibition (TGI) analysis results for BT474 tumors.
Figure 34 is a diagram that shows the analysis results of the cytotoxicity of linker conjugated Trastuzumab-NK cells against N87 gastric cancer cells.
Figure 35 is a diagram that shows the analysis results of the cytotoxicity linker conjugated Trastuzumab-NK cells against H2170 lung cancer cells.
Figure 36 is a diagram that shows the analysis results of the conjugating degree of linker conjugated Trastuzumab to NK cells under different UV irradiation energies.
Figure 37 is a diagram that shows the analysis results of the conjugating degree of different linker conjugated Trastuzumab to NK cells at different reaction times after UV irradiation.
Figure 38 is a diagram that shows the analysis of the conjugating degree of different linker conjugated Trastuzumab to NK at different antibody concentrations.
Figure 39 is a diagram that shows the analysis of the cytotoxicity results for different linker conjugated Trastuzumab at different antibody concentrations.

### Specific Embodiments

### [Modes for Carrying Out the Invention]

The embodiments of the present invention are described below. However, the present invention is not limited to the following embodiments. The medicaments for treatment or prevention and methods for treatment or prevention of the present invention may be administered to or applied to humans. In the present specification, the symbols "~" and "-" used to indicate a range are inclusive of the two endpoints, for example, "A~B" and "A-B" refer to a range that is A or more and B or less. In the present invention, the mean of the term "and/or" encompass appropriate combinations of "and" and "or." Specifically, "A, B, and/or C" includes the following seven variations: (i) A; (ii) B; (iii) C; (iv) A and B; (v) A and C; (vi) B and C; and (vii) A, B, and C.

In the present invention, the term "about" is used to refer to a value and includes the standard deviation of error (error) in the device or method used to determine the value. In certain embodiments, unless otherwise specified or clearly apparent from the context (for example, when the value exceeds 100% of the possible value), the term "about" refers to a numerical range that falls within a range of 25%, 20%, 19%, 18%, 17%, 16%, 15%, 14%, 13%, 12%, 11%, 10%, 9%, 8%, 7%, 6%, 5%, 4%, 3%, 2%, 1%, or less of the stated value in any direction (greater than or less than).

One embodiment of the present invention is a cross-linked complex molecule having the structure represented by the following Formula (A). 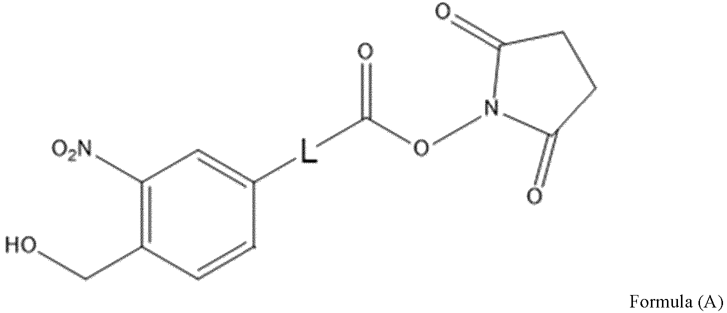 in which L is -O-(CH₂)ₘ-W-(CH₂)ₙ-, m and n are each independently integers from 0 to 10, and W is a single bond, or -NHCO-, or a substituted or unsubstituted polyethylene glycol (PEG). In some embodiments, W is polyethylene glycol with 1-4 units. Specifically, the crosslinked complex molecule may comprise a photo-reactive functional group moiety represented by the following Formula (A-1), and a targeting molecule conjugating moiety represented by the following Formula (A-2), with both moieties being linked through L.

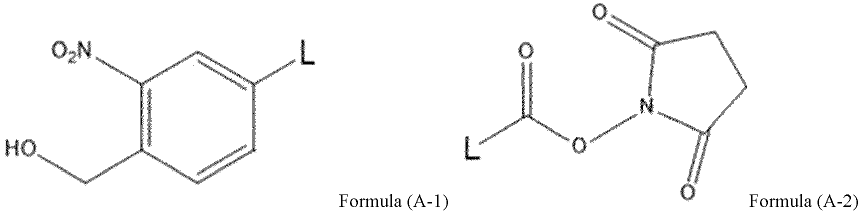

In some embodiments, the crosslinked complex molecule of the present invention is preferably formed by the conjugation of N-hydroxysuccinimide ester with 4-(4-(hydroxymethyl)-2-methoxy-5-nitrophenoxy)butanoic acid.

In some embodiments, the hydrogen on the benzene ring of the photo-reactive functional group moiety (A-1) of the crosslinked complex molecule may be further substituted with other optional substituents. The other optional substituents mentioned above may be, for example: halogen, hydroxyl group, sulfhydryl group, amino group, nitro group, cyano group, aldehyde group, ketone group, ester group, amide group, phosphonato group, phosphonate group, sulfo group, sulfonate group, sulfone group, sulfoxide group, aryl group, heteroaryl group, alkyl group, alkoxy group, alkylidene group, or modified alkyl group, but the present invention is not limited thereto. In some embodiments, the optional substituent is a methoxy group.

The crosslinked complex molecule of the present invention may undergo a photochemical cyclization reaction by irradiating the photo-reactive functional group moiety (A-1) with ultraviolet light (UV), thereby conjugating to an amino functional group (-NH₂) on the surface of a cell to form an indazolone moiety. In some embodiments, the ultraviolet light used to induce the photochemical cyclization reaction may have a wavelength of 300-400 nanometers, for example, ultraviolet light in the UVA band with a wavelength between 320-400 nanometers.

The cells that bind to the crosslinked complex molecule of the present invention are not particularly limited as long as their surface contains an amino functional group (-NH₂) that can react with the photo-reactive functional group moiety (A-1) to form an indazolone moiety. Examples of such cells may be: mesenchymal stem cells, blood cells, or bacterial cells. Mesenchymal stem cells may include, for example: adipose-derived mesenchymal stem cells, hematopoietic stem cells, bone marrow mesenchymal stem cells, umbilical cord mesenchymal stem cells, placental stem cells, or a combination thereof. Blood cells may include, for example: platelets, T cells, natural killer cells, dendritic cells, macrophages, granulocytes, or a combination thereof.

On the other hand, the targeting molecule conjugating moiety (A-2) of the crosslinked complex molecule of the present invention may first react with the targeting molecule through the N-hydroxysuccinimide ester group moiety, so that it is conjugated with the target molecule through an amide bond to form linker-targeting molecule complex. Subsequently, the linker-targeting molecule complex may further undergo photochemical cyclization of the photo-reactive functional group by irradiating ultraviolet light to conjugate to a cell, thereby forming a targeting molecule-cell complex. In some embodiments, the molar ratio of the targeting molecule to the linker used for forming the linker-targeting molecule complex is 1:1 to 1:15. In some embodiments, ultraviolet light irradiation may be carried out by irradiating the linker-target molecule complex for 5 to 30 seconds, followed by a standing period of 1 to 30 seconds in a cycle. In some embodiments, the cycles may be repeated 1 to 6 times. In some embodiments, after ultraviolet light irradiation, the linker-cell complex may react with the cell for at least 1 minute. It should be noted that the present invention does not particularly limit the order in which the cross-linked complex molecules bind to the cell and the targeting molecule, and thus the crosslinked complex molecule may first bind to the cell to form a linker-cell complex and then bind to the targeting molecule to form the targeting molecule-cell complex.

Formula (I) below shows an example of a targeting molecule-cell complex:

X-A₁-L-A₂-D Formula (I)

in which X is a cell; A1 is a substituted or unsubstituted indazolone moiety; L is O-(CH₂)ₘ-W-(CH₂)ₙ-, in which m and n are each independently integers from 0 to 10, and W is a single bond, or -NHCO-, or a substituted or unsubstituted polyethylene glycol with 1-4 units; A₂ is -CONH- or -COS-; and D is a targeting moiety.

Formulas (II) and (III) further show other examples of the targeting molecule-cell complex. From Formulas (II) and (III), it can be observed that the photoreactive functional group moiety of the crosslinked complex molecule binds to cell X to form the indazolone moiety, while the targeting moiety A₂ conjugates with the targeting molecule D. 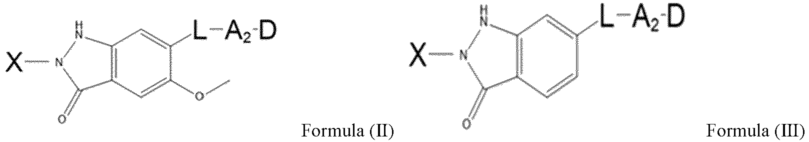 in which L is -O-(CH₂)ₘ-W-(CH₂)ₙ-, m and n are each independently integers from 0 to 10, and W is a single bond, or -NHCO-, or a substituted or unsubstituted polyethylene glycol (PEG); A₂ is -CONH- or -COS-; and D is a targeting moiety. In some embodiments, W is of polyethylene glycol with 1-4 units.

In the present invention, the term "targeting molecule" refers to a specific molecule that has antitumor efficacy and specifically inhibits tumors, and the targeting molecule can form a linker-targeting molecule complex with the crosslinked complex molecule of the present invention, or further form a targeting molecule-cell complex. On the other hand, the term "targeting moiety" refers to the portion derived from the targeting molecule in the linker-targeting molecule complex or the targeting molecule-cell complex. The targeting molecules include, but are not limited to, antibodies, proteins, peptides, small molecules, nucleic acid molecules, and those can be obtained from commercial suppliers and also can be manufactured by known methods. Examples of targeting molecules may include: antibodies such as Cetuximab, Bevacizumab, Panitumumab, Trastuzumab, Atezolizumab, Rituximab, Tositumomab, Ibritumomab, Alemtuzumab, Eprtuzumab, Natalizumab; small molecule drugs such as Gefitinib, Erlotinib, Afatinib; and aromatic amide derivatives with antitumor activity. In some embodiments, the targeting molecule/moiety may be an antigen-binding molecule. In some embodiments, the targeting moiety may be a small molecule, aptamer, peptide, antibody, or a combination thereof. In some embodiments, the targeting molecule may be Trastuzumab, Atezolizumab, or Rituximab. In some embodiments, the antibody may be a single-chain variable fragment (scFv), a fragment antigen-binding (Fab) fragment, or a full-length antibody. In some embodiments, the targeting moiety may have an amino group (NH₂ group) or a sulfhydryl group.

The targeting molecule-cell complex of the present invention may be used in treatment and prevention of cancer, and may also be used in the treatment of autoimmune diseases. In some embodiments, the targeting molecule-cell complex may be used to promote cell homing. In the present invention, the term "treatment of cancer" refers to reducing the number of cancer cells in a subject, inhibiting the proliferation of cancer cells, reducing tumor volume, reducing tumor weight, inhibiting cancer cell metastasis, or alleviating various symptoms caused by cancer. The term "prevention of cancer" refers to reducing the number of cancer cells in a subject, inhibiting cancer cell proliferation, reducing tumor volume, reducing tumor weight, inhibiting metastasis of cancer cells, or alleviating various symptoms caused by cancer. The term "prevention of cancer" refers to preventing an increase in cancer cell number due to the regrowth of previously reduced cancer cells, preventing the regrowth of proliferation-inhibited cancer cells, or preventing an increase in tumor volume or weight after a reduction. In the present invention, the term "cancer" includes solid tumors and hematological tumors, and regardless of the type, all are composed of abnormally proliferating cells that grow uncontrollably. Solid tumors are formed by one or more tumors, while hematological tumors circulate throughout the body via the bloodstream. In the present invention, the term "targeting molecule-cell complex that can be used in the treatment and/or prevention of a cancer" refers to a targeting molecule-cell complex that can be used to treat and/or prevent cancers such as solid tumors and/or hematological tumors. In the present invention, in some embodiments, examples of cancers include, but are not limited to: lung cancer, esophageal cancer, gastric cancer, colorectal cancer, uterine cancer, ovarian cancer, ampullary cancer, pancreatic cancer, pancreatic cancer, bladder cancer, thyroid cancer, skin cancer, head and neck cancers, liver cancer, breast cancer, colon cancer, renal cancer, muscle tumors, prostate cancer, testicular cancer, cholangiocarcinoma, Merkel cell carcinoma (Merkel cell carcinoma), schwannoma, adrenal cancer, anal cancer, central nervous system tumors, neuroendocrine tumors, penile cancer, pleural tumors, salivary gland tumors, vulvar cancer, thymoma, and pediatric cancers (such as Wilms tumor, neuroblastoma, sarcoma, hepatoblastoma, and germ cell tumor), leukemia, malignant lymphoma, and multiple myeloma. In some embodiments, the cancer is breast cancer, colon cancer, pancreatic cancer, lung cancer, or gastric cancer.

### [Examples]

The specific details of the present invention will be further explained through several examples below, but the present invention is not limited to these embodiments. The materials, agents, and other substances used in each embodiment can be obtained from commercial suppliers and also can be manufactured by known methods.

### Example 1 [Method of Preparing Linker Formula A]

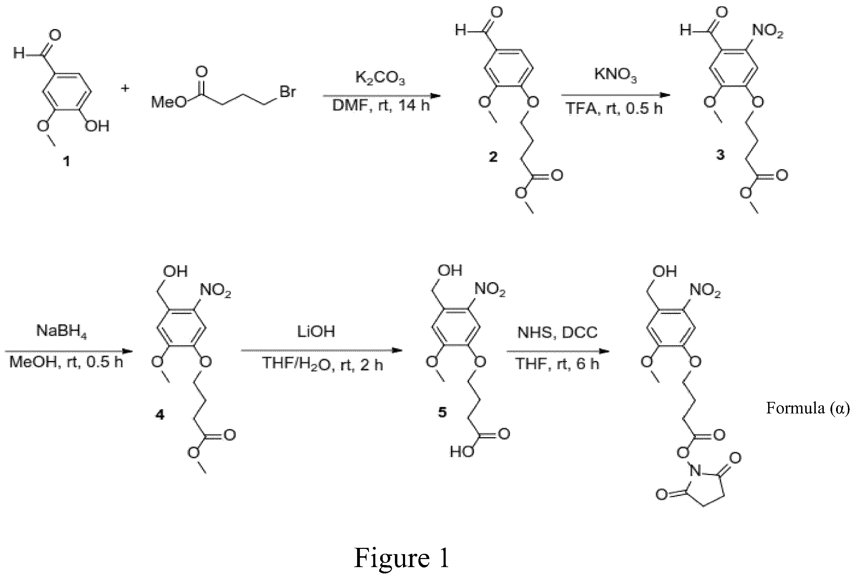

Figure 1 shows a preparation flow process of linker formula α as shown below.

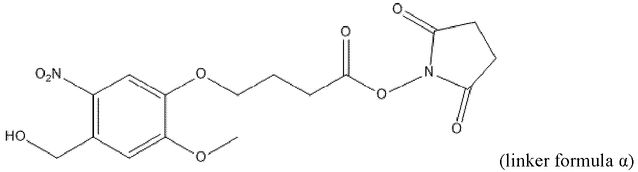

The following will refer to Figure 1 to describe the detailed synthesis steps of linker formula α.

### Step 1:

20 g, 0.13 mol of 4-hydroxy-3-methoxybenzaldehyde (Compound 1) was dissolved in 150 mL of dimethylformamide (DMF), then 27.4 g, 0.2 mol of K₂CO₃ and 25 g, 0.14 mol of methyl 4-bromobutanoate were added, and the mixture was stirred at room temperature for 14 hours. Then, water was added to the resulting mixture at 0°C, and the resulting solid was filtered to obtain 30 g, 90% of Compound 2 as a white solid.

### Step 2:

24.8 g, 98 mmol of Compound 2 was prepared in 50 mL of trifluoroacetic acid (TFA), then 12.5 g, 0.12 mol of KNO₃ was added to the Compound 2 solution at 0°C, and the mixture was stirred at room temperature for 0.5 hours. Then, the mixture was concentrated to dryness and the crude product was dissolved in 500 mL of ethyl acetate, and the solution was neutralized with saturated NaHCO₃ aqueous solution to pH 7. The organic layer was sequentially washed with water and saline, and dried with Na₂SO₄. Then, the mixture was filtered and concentrated to dryness to obtain 29 g, 99% of Compound 3 as a yellow solid.

### Step 3:

29 g, 98 mmol of Compound 3 was prepared in 500 mL of methanol/125 mL of tetrahydrofuran (THF), then 5.54 g, 0.15 mol of NaBH₄ was slowly added to the Compound 3 solution at 0°C. Then, the mixture was stirred at room temperature for 0.5 hours and concentrated to dryness. 200 mL of ether and 200 mL of water were added to the crude product and filtered to obtain 29 g, 99% of Compound 4 as a light yellow solid.

### Step 4:

29 g, 97 mmol of Compound 4 was prepared in 250 mL of THF, then the Compound 4 solution was added to a LiOH solution formed by dissolving 7 g of LiOH in 125 mL of water, and the mixture was stirred at room temperature for 2 hours. Then, the mixture was concentrated to remove the THF. The solution was acidified with 1 N HCl to pH = 5, and filtered to obtain 19.8 g, 72% of Compound 5 as a yellow solid.

### Step 5:

19.8 g, 70 mmol of Compound 5 was prepared in 200 mL of THF, then 17.2 g, 83 mmol of N,N'-dicyclohexylcarbodiimide (DCC) and 9.6 g, 83 mmol of N-hydroxysuccinimide (NHS) were added to the Compound 5 solution, and the mixture was stirred at room temperature for 6 hours. Then, the solid was filtered and the filtrate was concentrated to dryness, then the crude product was purified by column chromatography (EA/Hex = 3/5) to obtain 9.4 g, 35% of the hard and yellow linker formula α.

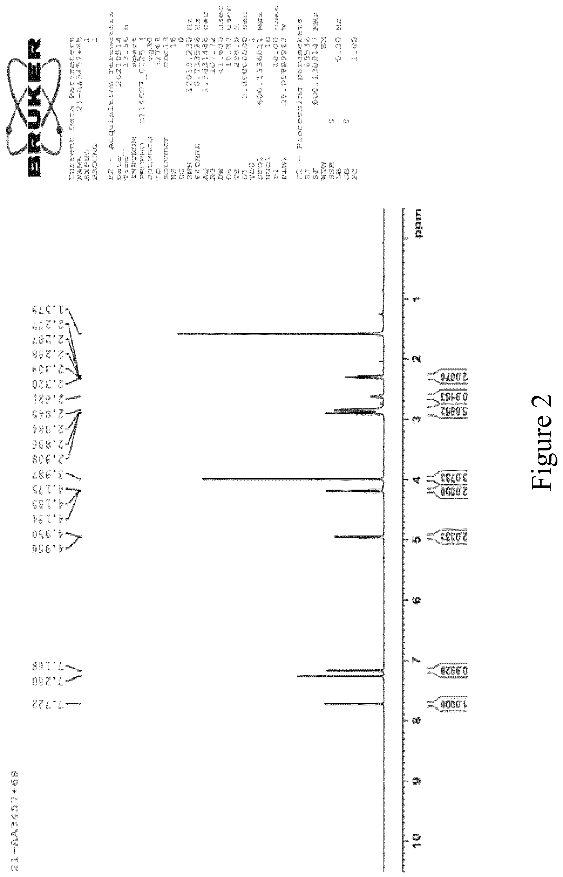

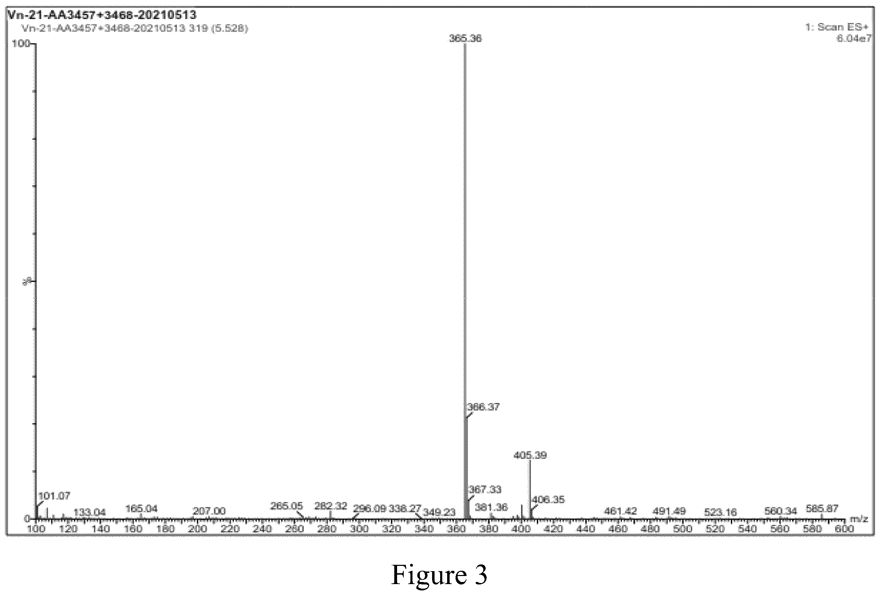

Figure 2 shows the nuclear magnetic resonance (NMR) analysis result of linker compound α, the analysis data was as follows: 1H NMR (600 MHz, CDCl3) δ 7.72 (s, 1H), 7.17 (s, 1H), 4.95 (d, J = 3.6 Hz, 2H), 4.19 (t, J = 5.9 Hz, 2H), 3.99 (s, 3H), 2.90 (t, J = 7.2 Hz, 2H), 2.85 (br s, 4H), 2.62 (br s, 1H), 2.30 (quin, J = 6.6 Hz, 2H)).Figure 3 shows the liquid chromatography-tandem mass spectrometry (LC-MS/MS) analysis result of linker compound α.

### Example 2 [Method of Preparing Linker Formula β]

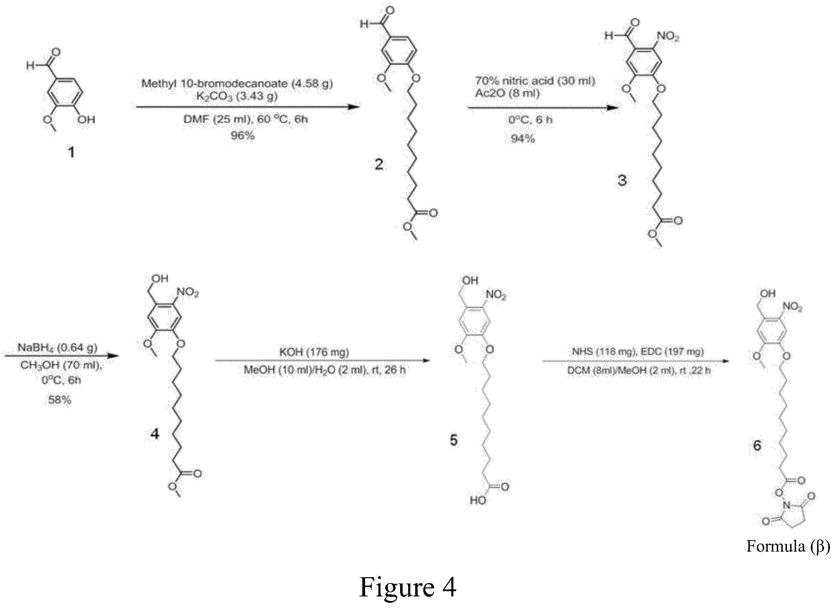

Figure 4 shows a preparation flow process of linker formula β as shown below.

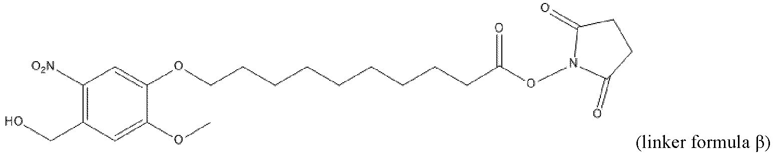

The following referred to Figure 4 to describe the detailed synthesis steps of linker formula β.

### Step 1:

2.5 g, 16.4 mmol of 4-hydroxy-3-methoxybenzaldehyde (Compound 1) was dissolved in 25 mL of DMF, then 3.43 g, 24.8 mmol of K₂CO₃ and 4.58 g, 17.27 mmol of methyl 4-bromobutanoate were added, and the mixture was stirred at 60°C for 6 hours. Then, water was added to the mixture at 0°C, and the resulting solid was filtered to obtain 5.3 g, 96% of Compound 2 as a white solid.

### Step 2:

3 g, 8.18 mmol of Compound 2 was prepared in 30 mL of 70% nitric acid/8 mL of acetic anhydride, then the mixture was reacted at 0°C for 6 hours. Then, the mixture was concentrated to dryness and the crude product was dissolved in 50 mL of ethyl acetate, then the solution was neutralized with saturated NaHCO₃ aqueous solution to pH = 7. The organic layer was sequentially washed with water and saline, and dried with Na₂SO₄. Then, the mixture was filtered and concentrated to dryness to obtain 3.2 g, 94% of Compound 3 as a yellow solid.

### Step 3:

3.2 g, 8.39 mmol of Compound 3 was prepared in 70 mL of CH₃OH, then 0.64 g, 16.91 mmol of NaBH₄ was slowly added to the Compound 3 solution at 0°C. Then, the mixture was reacted at 0°C for 6 hours, and concentrated to dryness. 30 mL of ether and 30 mL of water were added to the crude product, and filtered to obtain 1.86 g, 58% of Compound 4 as a light yellow solid.

### Step 4:

200 mg, 0.52 mmol of Compound 4 was prepared in 10 mL of methanol/2 mL of water, then the Compound 4 solution was added to a KOH solution prepared with 3.13 mmol of KOH, and the mixture was stirred at room temperature for 26 hours. Then, the mixture was filtered to obtain 190 mg, 98% of Compound 5 as a yellow solid.

### Step 5:

190 mg, 0.51 mmol of Compound 5 was prepared in 8 mL of dichloromethane (DCM)/2 mL of methanol, then 197 mg, 1.99 mmol of 1-Ethyl-3-(3-dimethylaminopropyl)carbodiimide (EDC) and 118 mg, 1.02 mmol of NHS were added to the Compound 5 solution, and the mixture was stirred at room temperature for 22 hours. Then, the solid was filtered and the filtrate was concentrated to dryness, then the crude product was purified by column chromatography (EA/Hex=3/5) to obtain 63 mg, 26% of the hard and yellow linker formula β.

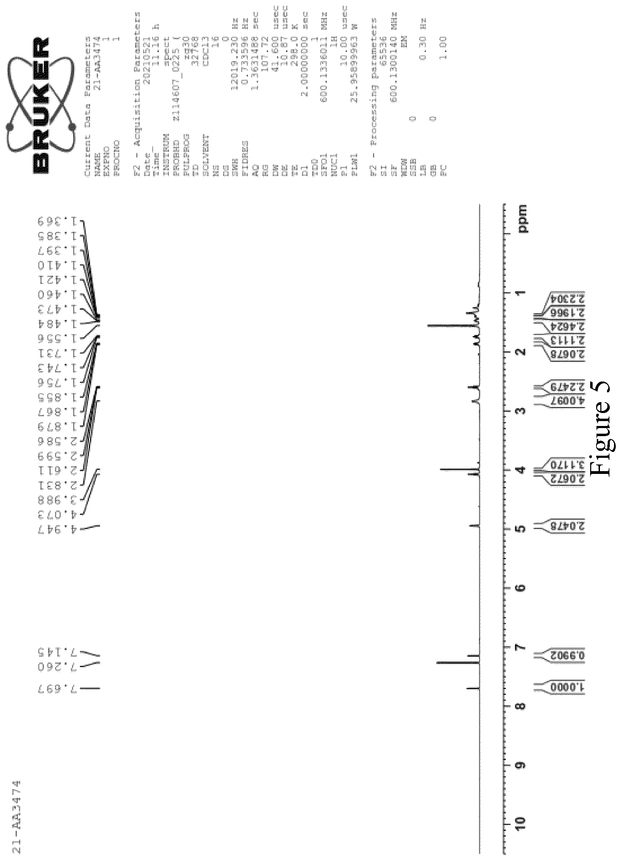

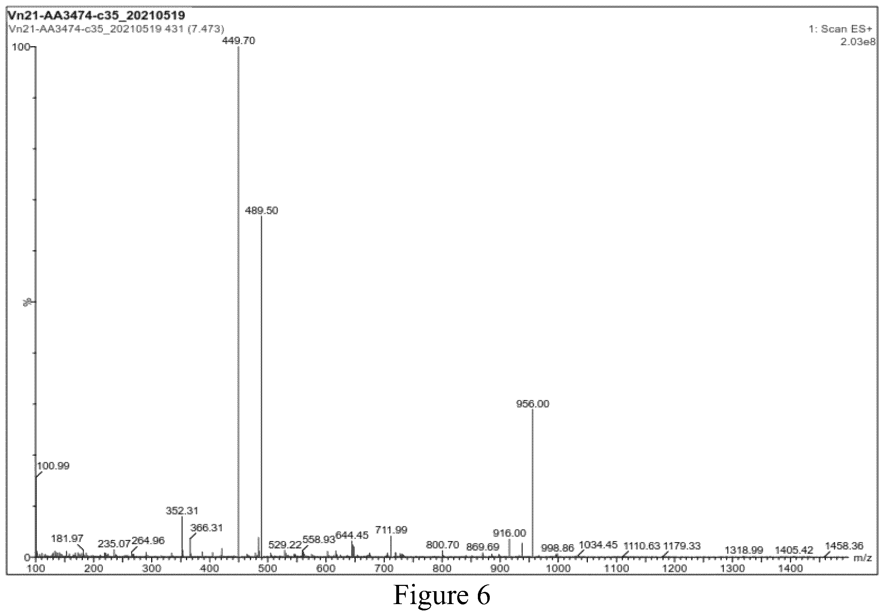

Figure 5 shows an NMR analysis result of linker compound β, the analysis data was as follows: 1H NMR (600 MHz, CDCl3) δ 7.71 (s, 1H), 7.16 (s, 1H), 4.96 (d, J = 3.7 Hz, 2H), 4.09 (t, J = 6.8 Hz, 2H), 4.00 (s, 3H), 2.85 (d, J = 7.4 Hz, 4H), 2.61 (t, J = 7.5 Hz, 3H), 1.88 (h, J = 7.3 Hz, 2H), 1.76 (p, J = 7.6 Hz, 2H), 1.48 (h, J = 7.5 Hz, 2H), 1.45 - 1.35 (m, 6H), 1.35 (s, 3H), 1.30 - 1.25 (m, 2H), 0.92 - 0.84 (m, 1H).Figure 6 shows an LC-MS/MS analysis result of linker compound β

### Example 3 [Method of Preparing Linker Formula γ]

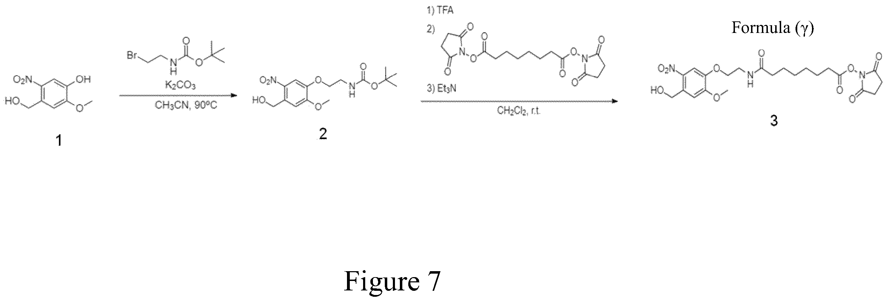

Figure 7 shows a preparation flow process of linker formula γ as shown below.

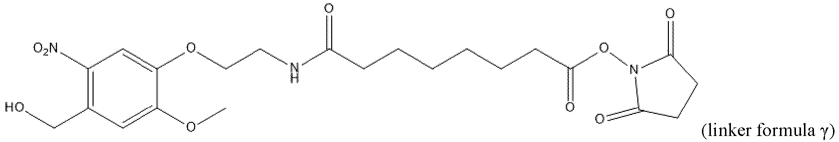

The following referred to Figure 7 to describe the detailed synthesis steps of linker formula γ.

### Step 1:

500 mg, 2.51 mmol of 4-(hydroxymethyl)-2-methoxy-5-nitrophenol (Compound 1) was dissolved in 13 mL of CH₃CN, then 1.39 g, 10.04 mmol of K₂CO₃ and 1.12 g, 5.02 mmol of N-Boc-2-bromoethyl-amine were added, and the mixture was stirred under Ar gas at 90°C for 18 hours. Then, the resulting solid was filtered off to obtain a concentrated liquid, and it was purified by column chromatography to obtain 234 mg, 27% of Compound 2 as a viscous liquid.

### Step 2:

210 mg, 0.61 mmol of Compound 2 was prepared in 4 mL of CH₂Cl₂, and 0.4 mL of TFA was added to react at room temperature for 2 hours. Then, the mixture was concentrated to dryness using a rotary evaporator, the crude product was dissolved in 4 mL of dry CH₂Cl₂, and 314 mg, 0.85 mmol of disuccinimidyl suberate (DSS) and 0.25 mL, 1.83 mmol of triethylamine (Et3N) were sequentially added, and the mixture was reacted at room temperature for 2 hours. Then, the organic layer was removed using a rotary evaporator and the product was purified by column chromatography to obtain 32 mg, 15% of linker formula γ as a viscous liquid.

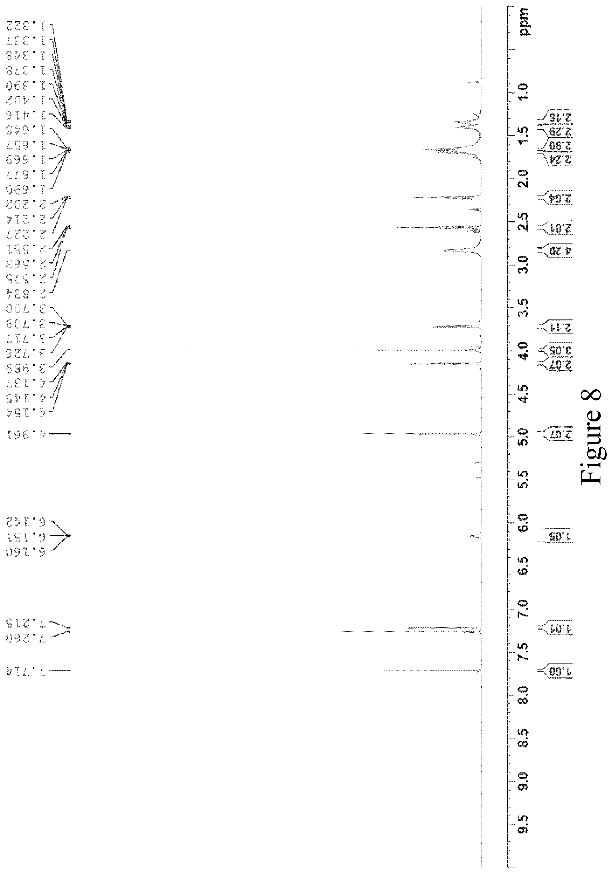

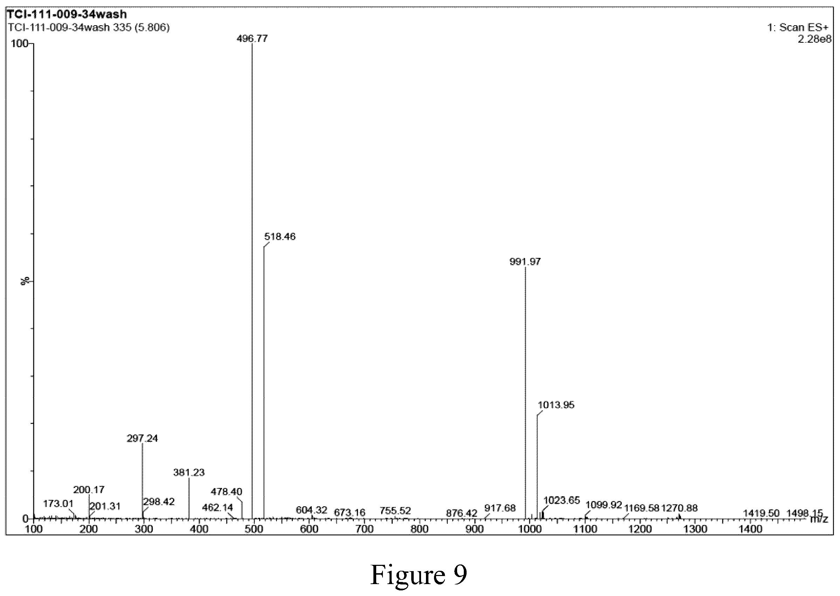

Figure 8 shows an NMR analysis result of linker formula γ, and the analysis data was as follows: 1H NMR (600 MHz, CDCl3) δ 7.73 (s, 1H), 7.23 (s, 1H), 6.17 (d, J = 6.1 Hz, 1H), 4.98 (s, 2H), 4.16 (t, J = 5.2 Hz, 2H), 4.01 (s, 3H), 3.73 (q, J = 5.4 Hz, 2H), 2.85 (s, 3H), 2.65 - 2.55 (m, 3H), 2.23 (t, J = 7.4 Hz, 2H), 1.81 - 1.63 (m, 9H), 1.49 - 1.30 (m, 6H).Figure 9 shows the LC-MS/MS analysis result of linker formula γ.

### Example 4 [Method of Preparing Linker Formula δ]

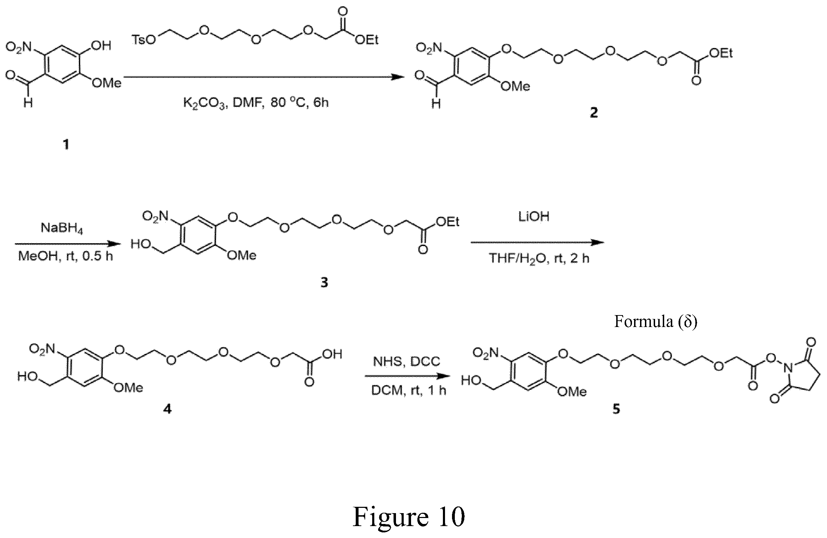

Figure 10 shows a preparation flow process of linker formula δ as shown below. As shown in Figure 10, compared to linker formulas α, β, and γ, linker formula δ possessed a polyethylene glycol (PEG) structure.

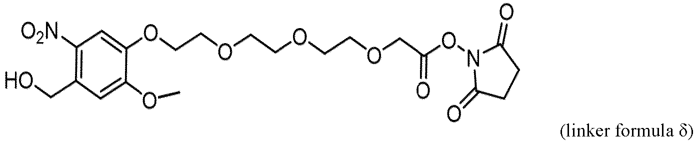

The following referred to Figure 10 to describe the detailed synthesis steps of linker formula δ.

### Step 1:

0.5 g, 2.5 mmol of 4-hydroxy-5-methoxy-2-nitrobenzaldehyde (Compound 1) was dissolved in 5 mL of DMF, then 0.53 g, 3.8 mmol of K₂CO₃ and 1.09 g, 2.8 mmol of ethyl 2-(2-(2-(2-(tosyloxy)ethoxy)ethoxy)ethoxy)acetate were added at room temperature, and the mixture was stirred at 80°C for 6 hours. Then, 20 mL of water was added to the resulting mixture at 0°C, and the obtained solid was filtered to obtain 0.88 g, 84% of Compound 2 as a yellow solid.

### Step 2:

0.88 g, 2 mmol of Compound 2 was prepared in 15 mL of methanol/5 mL of THF, and NaBH₄ was slowly added, then the mixture was stirred at room temperature for 0.5 hours. The resulting mixture was diluted with 100 mL of ethyl acetate, and the organic layer was sequentially washed with 100 mL of water and 100 mL of saline, then dried with Na₂SO₄. The resulting mixture was filtered and concentrated to dryness to obtain 0.87 g, 98% of Compound 3 as a yellow solid.

### Step 3:

0.87 g, 2 mmol of Compound 3 was prepared in 8 mL of THF, and 3 mL of aqueous solution containing 0.64 g, 16.91 mmol of LiOH was slowly added at 0°C, and after the addition was complete, the mixture was stirred at room temperature for 2 hours. The mixture was concentrated to remove THF, then acidified with 1 N HCl to pH = 5. The resulting solid was filtered to obtain 0.8 g, 99% of Compound 4 as a light yellow solid.

### Step 4:

200 mg, 0.5 mmol of Compound 4 was prepared in 3 mL of THF, then 120 mg, 0.6 mmol of EDC and 65 mg, 0.6 mmol of NHS were added to the Compound 4 solution, and the mixture was stirred at room temperature for 1 hour. The crude product was purified by column chromatography (Merck silica gel, 1.09385.9025) to obtain 78 mg, 31% of linker formula δ as a yellow oily product.

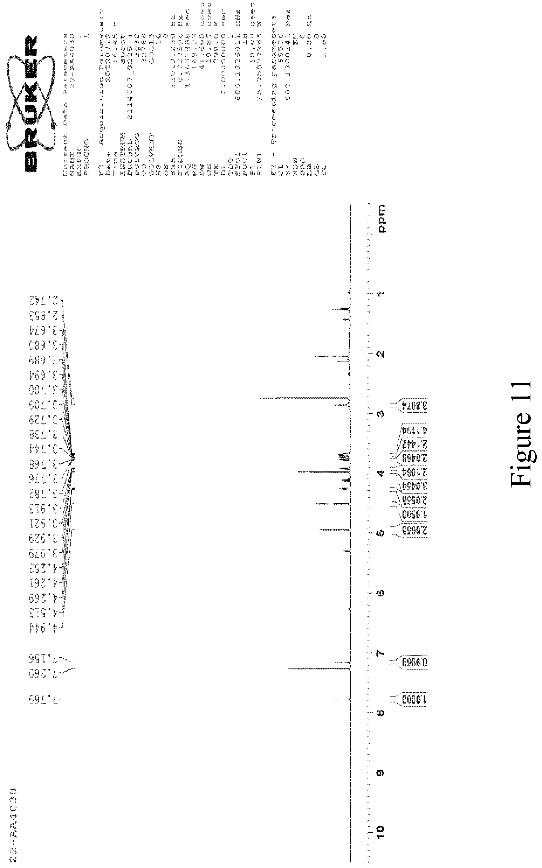

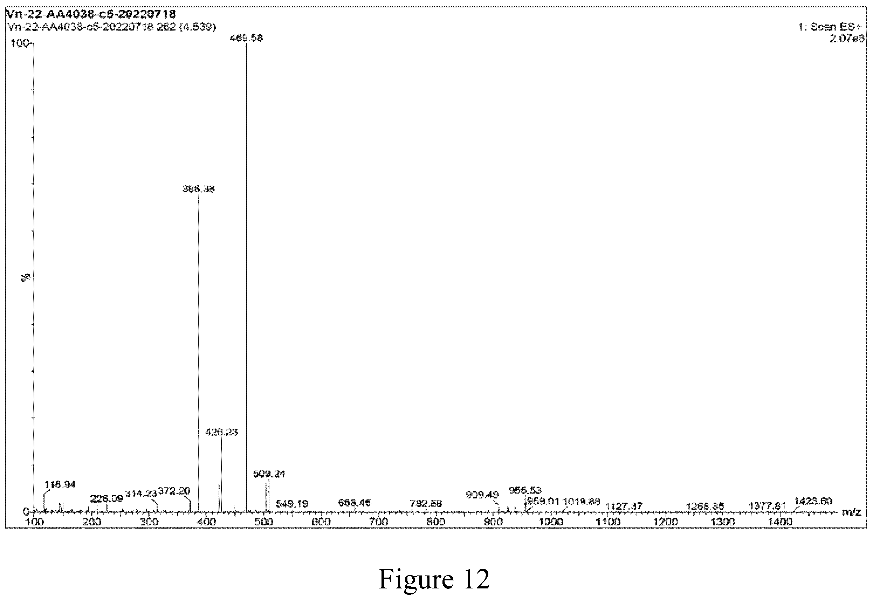

Figure 11 shows an NMR analysis result of linker formula δ, and the analysis data was as follows: 1HNMR (600 MHz, CDCl3) δ 7.79 (s, 1H), 7.17 (s, 1H), 4.96 (s, 2H), 4.53 (s, 2H), 4.28 (dd, J = 5.5, 4.1 Hz, 2H), 4.00 (s, 3H), 3.96 - 3.91 (m, 2H), 3.79 (dd, J = 5.8, 3.2 Hz, 2H), 3.77 - 3.66 (m, 7H), 2.87 (s, 4H). Figure 12 shows an LC-MS/MS analysis result of linker formula

### Example 5 [Sodium Dodecyl-Sulfate Polyacrylamide Gel Electrophoresis (SDS-PAGE) Analysis of Different Hydrocarbon Chain Linker Conjugated Trastuzumab]

The following describes the SDS-PAGE analysis steps of hydrocarbon chain linker conjugated Trastuzumab.

### Step 1:

2.1 mg of linker formula α from Example 1 was taken and mixed with 551 µL of dimethyl sulfoxide (DMSO) to prepare a 10 mM linker formula α solution; 2.7 mg of linker formula β from Example 2 was taken and mixed with 579 µL of DMSO solvent to prepare a 10 mM linker formula β solution; 1.1 mg of linker formula γ from Example 3 was taken and mixed with 222 µL of DMSO solvent to prepare a 10 mM linker formula γ solution; 2 mg of commercially available DSS (manufactured by Thermo Scientific) was taken and mixed with 544 µL of DMSO solvent to prepare a 10 mM DSS solution.

### Step 2:

Four tubes of 5 mL (2 mg/mL) Herceptin antibody (Roche, Trastuzumab) were taken, and were separately added to 67.55 µL of 10 mM linker formula α solution, 67.55 µL of 10 mM linker formula β solution, 67.55 µL of 10 mM linker formula γ solution, and 67.55 µL of 10 mM DSS solution from Step 1 and mixed. The mixtures were then reacted at room temperature for 30 minutes to generate the 1-T solution, 2-T solution, 3-T solution, and DSS-T solution, respectively.

### Step 3:

The solutions of Trastuzumab conjugated with different linkers were placed into Amicon^{®} Ultra Centrifugal Filters, then 11 mL of phosphate buffered saline (PBS) was added, and centrifuged at 2500 g for 30 minutes at 4-8°C. The waste liquid from the bottom of the Amicon^{®} Ultra Centrifugal Filter was removed, 6 mL of PBS was added from the top, and centrifuged again at 2500 g for 30 minutes at 4-8°C. The waste liquid was removed from the bottom of the filter, and the absorbance at 280 nm (OD280) was measured to calculate the concentration. The antibody concentration was adjusted to 5 mg/mL using PBS, and mixed with 62.5% glycerol at a volume ratio of 1 (antibody):4 (glycerol), then stored at -20°C, such that the final antibody concentration of 1-T, 2-T, 3-T, and DSS-T solutions was 1 mg/mL for subsequent use.

### Step 4:

5 µg of Trastuzumab and different hydrocarbon chain linker conjugated Trastuzumab were taken, and respectively mixed with 10 µL of non-reducing sample buffer (3% SDS, 0.3% bromophenol blue, 15% glycerol, and 188 mM Tris-HCl at pH 6.8), loaded onto a 10% SDS-polyacrylamide gel, and electrophoresis was carried out (Mini-PROTEAN^{®} Tetra Vertical Electrophoresis Cell/1658004/BIO-RAD) in 1× Tris-glycine SDS running buffer (1 L, 3.03 g Tris, 14.4 g glycine, and 2% SDS). The electrophoresis conditions were: run at constant voltage of 100 V for about 20 minutes until the dye front entered the stacking gel, then switch to 160 V constant voltage, and stop the electrophoresis when the dye front reached the bottom of the gel, for a total of about 80 minutes.

### Step 5:

The SDS-polyacrylamide gel was taken out and stained in staining solution (0.08% w/v Coomassie Brilliant Blue R-250, 50% (v/v) methanol, and 10% (v/v) acetic acid) for about 30 minutes, then the staining solution was poured off and destaining solution (50% (v/v) methanol, 10% (v/v) acetic acid) was added, and the solution was slowly shaken and replaced as needed until the gel background became clear.

### Experimental Results:

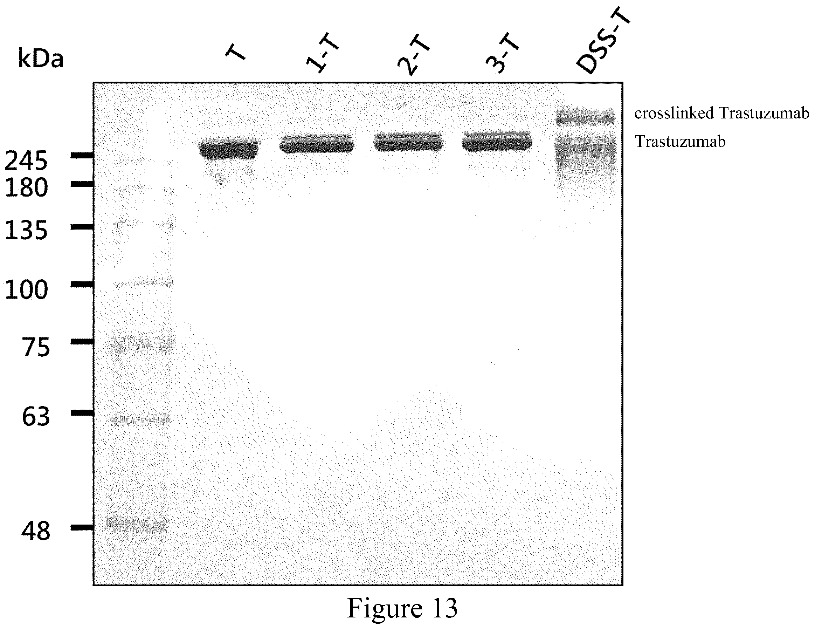

Figure 13 shows the result of SDS-PAGE analysis of different hydrocarbon chain linker conjugated Trastuzumab. As shown in Figure 13, compared with the control group Trastuzumab (T), the commercially available control DSS-T exhibited increased molecular weight due to antibody crosslinking. Therefore, in non-reducing SDS-PAGE analysis, DSS-T also appeared at a higher molecular weight position (>245 kDa). Such antibody crosslinking might have affected subsequent conjugating to cells. However, in the experimental groups: 1-T, 2-T, and 3-T, since no antibody crosslinking occurred, the molecular weights of the experimental groups observed on SDS-PAGE matched that of the control group Trastuzumab (T).

### Example 6 [Cellular Stability Test of Linker Conjugated Trastuzumab]

The following describes the steps for comparing the conjugating degree of different hydrocarbon chain linker conjugated Trastuzumab to adipose-derived mesenchymal stem cells (ADMSC).

### Step 1:

1 mL of frozen human adipose-derived mesenchymal stem cells (ADMSC) (TCI GENE; Lot.no. 20210816) and 10mL of PBS were transferred to a 15mL centrifuge tube, centrifuged at 400g for 5 minutes, and transferred to 10mL of mesenchymal stem cell (MSC) culture medium (Minimum Essential Medium α (α MEM)/5% EliteGro (human platelet lysate)/1ng/mL fibroblast growth factor β (FGF-β)/20 ng/mL platelet-derived growth factor BB (PDGF-BB)), and the resulting suspension was transferred to a 10 cm culture dish and incubated at 37°C, 5% CO₂ for three days.

The ADMSC cells were treated with CTS^{™} TrypLE^{™} Select enzyme (Gibco^{™}, A1285901) and transferred to a 15 mL centrifuge tube, centrifuged at 400g for 5 minutes at 4-8°C, and the supernatant was removed, then the ADMSC cells were washed twice with PBS, and finally the ADMSC cells were resuspended in PBS, and the cell concentration was adjusted to 2×10⁶ cells/mL using PBS. Finally, 1 mL of the resulting solution was distributed into each of six tubes and labeled as control group, comparison group, and experimental groups (1), (2), (3), and (4), respectively.

### Step 2:

### [Preparation of Control Group, Comparison Group, and Experimental Groups]

The control group was formed by adding 1 mL of PBS and 1 mL of ADMSC cell solution; the comparison group was prepared by taking 940 µL of PBS and adding 60 µL of 1 mg/mL Trastuzumab solution and DSS-T solution, respectively; the experimental groups were prepared by taking 940 µL of PBS and adding 60 µL of 1 mg/mL of 1-T, 2-T, and 3-T solutions, respectively. Then, the solutions were then uniformly irradiated with UV at a wavelength of 365 nm for 10 seconds, followed by a standing period of 10 seconds each time, repeated twice in total, then 1 mL of MSC cell solution from Step 1 was added, resulting in a final Trastuzumab antibody concentration of 30 µg/mL and a final concentration of different hydrocarbon chain linker conjugated Trastuzumab of 30 µg/mL. Then, the mixtures of the control group, comparison group, and experimental groups were then allowed to stand at 4°C for 10 minutes.

### Step 3:

The mixtures from Step 2 were centrifuged at 400g for 5 minutes at 4-8°C, the supernatant was removed, then the cells were washed twice with 10 mL of PBS, and finally resuspended in PBS, and the cell concentration was adjusted to 1×10⁶ cells/mL for subsequent experiments.

### Step 4:

200 µL of the solution of each group from Step 3 was taken and transferred into a 1.5 mL test tube (Eppendorf), and after Goat F(ab')2 Anti-Human IgG-(Fab')2 (PE) (abcam, cat. no. ab98606) was diluted with PBS at a volume ratio of 1:100, 200 µL of this dilution was taken and added to solutions of each group, and reacted on ice for 15 minutes. Then, 800 µL of PBS was added, and the solutions were centrifuged at 400g for 5 minutes at 4-8°C, then the supernatant was removed, then the cells were washed twice with 1 mL of PBS, the supernatant was removed, and the cells were resuspended in 200 µL of PBS for each group, and finally analyzed by flow cytometry (fluorescence activated cell sorting, FACS).

### Experimental Results:

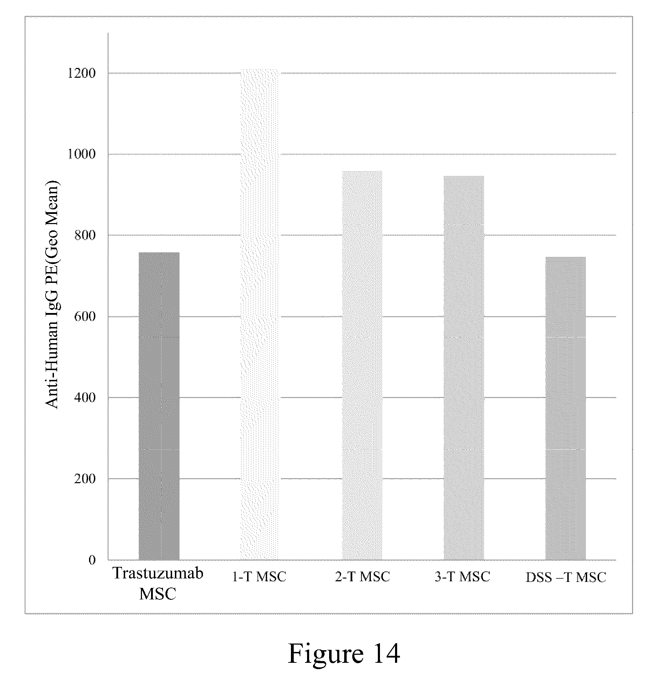

Goat F(ab')2 Anti-Human IgG-(Fab')2 (PE) was used for antibody staining on the ADMSC cell membrane, followed by flow cytometry analysis of the PE signal to obtain the strength of antibody conjugation to ADMSC. Figure 14 shows a comparative test result of the conjugating degree of different hydrocarbon chain linker conjugated Trastuzumab to ADMSC. From Figure 14, it was observed that the experimental groups 1-T MSC, 2-T MSC, and 3-T MSC successfully conjugated to NK cells compared to the control group Trastuzumab MSC and the comparison group DSS-T MSC, in which 1-T demonstrating the strongest conjugating ability to ADMSC.

### Example 7: [Analysis of the Conjugating Degree of Different Hydrocarbon Chain Linker Conjugated Trastuzumab to Natural Killer NK cells]

The following describes the steps for comparing the conjugating degree of different hydrocarbon chain linker conjugated Trastuzumab to NK cells.

### Step 1:

1 mL of Frozen Human NK cells (TCI GENE; Lot.no. 20210816) and 10 mL of PBS were transferred to a 15 mL centrifuge tube, centrifuged and washed at 400g for 5 minutes, then the NK cell concentration was adjusted to 5×10⁵ cells/mL using NK cell culture medium (Gibco Roswell Park Memorial Institute Medium (RPMI), 5% EliteGro, 500 IU/mL rhIL-2) and transferred to a T75 culture flask for incubation. After incubating at 37°C, 5% CO₂ for one day, the NK cells were washed and the buffer was replaced using the following methods (A) or (B):
(A) The NK cells were transferred to a 15 mL centrifuge tube, centrifuged at 400g for 5 minutes at 4-8°C, the supernatant was removed, then the NK cells were washed with 10 mL PBS twice, and the NK cells were resuspended in PBS and the NK cell concentration was adjusted to 2×10⁶ cells/mL using PBS.
(B) The NK cells were transferred to a 50 mL centrifuge tube, centrifuged at 400g for 5 minutes at 4-8°C, the supernatant was removed, then the NK cells were washed with 50 mL PBS once, and the NK cells were resuspended in PBS and the NK cell concentration was adjusted to 2×10⁶ cells/mL using PBS.

Then, 1 mL of the resulting solution was distributed into each of the 6 tubes and labeled as control group, comparison group, and experimental groups (1), (2), (3), and (4), respectively.

### Step 2:

### [Preparation of Control Group, Comparison Group, and Experimental Groups]

The control group was a mixture formed by adding 1 mL of PBS and 1 mL of NK cell solution; the comparison group was prepared by taking 940 µL of PBS and adding 60 µL of 1 mg/mL Trastuzumab solution and DSS-T solution, respectively; the experimental groups were prepared by taking 940 µL of PBS and adding 60 µL of 1 mg/mL of 1-T, 2-T, and 3-T solutions, respectively. Then, the resulting solution was mixed with 1 mL of NK cell solution from Step 1 at a volume of 1:1 to make the final Trastuzumab concentration of 30 µg/mL and the NK cell concentration of 1×10⁶ cells/mL. Finally, the solutions were uniformly irradiated with UV at a wavelength of 365 nm with 400 mJ/cm², and the mixtures of the control group, comparison group, and experimental groups were allowed to stand at 4°C for 10 minutes.

### Step 3:

The mixtures from Step 2 were divided into the following two groups for subsequent experiments:
(A) For small-scale cell test: The mixtures from Step 2 were transferred to a 15 mL centrifuge tube and an equal volume of PBS/1%HSA solution was added. After the resulting solutions were centrifuged at 400g for 5 minutes at 4-8°C, the supernatant was removed, then the NK cells were washed with 10 mL PBS/1%HSA twice, and the NK cells were resuspended in the solution required for the experiment and the NK cell concentration was adjusted to 2×10⁶ cells/mL.
(B) For large-scale animal test: The mixtures from Step 2 were transferred to a 50 mL centrifuge tube and an equal volume of PBS/1%HSA solution was added. After the resulting solutions were centrifuged at 400g for 5 minutes at 4-8°C, the supernatant was removed, then the NK cells were washed with 50 mL PBS/1%HSA once, and the NK cells were resuspended in the solution required for the experiment and the NK cell concentration was adjusted to 2×10⁶ cells/mL.

### Step 4:

After 200 µL of solutions of each group from Step 3 was taken to 1.5 mL test tube (Eppendorf), and Goat F(ab')2 Anti-Human IgG-(Fab')2 (PE) (abcam, cat.no. ab98606) was diluted in PBS at a volume ratio of 1:100, 200 µL of this dilution was taken and added to solutions of each groups from Step 3, along with 5 µL of CD16 monoclonal antibody (eBioscience^{™}. 17-0168-42), and the mixtures were incubated at 4°C for 30 minutes. 800 µL PBS was added, and the solutions were centrifuged at 400g for 5 minutes at 4-8°C, the supernatant was removed, then the cells were washed with 1 mL PBS twice, the supernatant was removed again, and the cells were resuspended in 200 µL PBS for each group, and finally analyzed by flow cytometry (fluorescence activated cell sorting, FACS).

### Experimental Results:

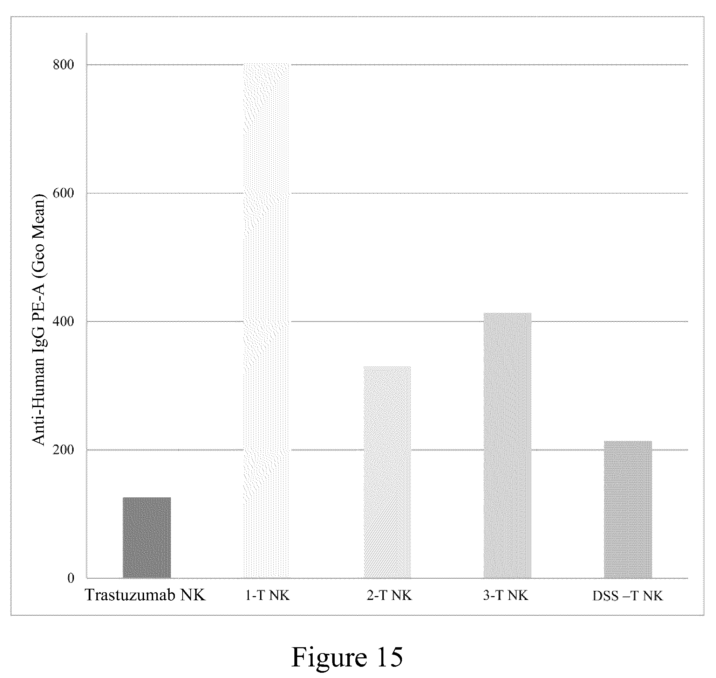

Goat F(ab')2 Anti-Human IgG-(Fab')2 (PE) was used for antibody staining on the NK cell membrane, followed by flow cytometry analysis of the PE signal to obtain the strength of Trastuzumab conjugating to NK cells. Figure 15 shows a comparative test result of the conjugating degree of different hydrocarbon chain linker conjugated Trastuzumab to NK cells. From Figure 15, it can be observed that, compared to the control group Trastuzumab NK and the commercially available DSS-T NK cells, the experimental groups 1-T NK, 2-T NK, and 3-T NK were successfully conjugated to NK cells, in which 1-T showing the strongest conjugating ability to the surface of NK cells. From Examples 6 and 7, it can be observed that among the four hydrocarbon chain linkers, the antibody prepared with linker formula α showed the best conjugating ability to cells.

### Example 8 [Analysis of the Cytotoxicity of Different Hydrocarbon Chain linker conjugated Trastuzumab-NK cells against BT474 breast cancer cells]

The following describes the steps for comparing the cytotoxicity of different hydrocarbon chain linker conjugated Trastuzumab-NK cells against BT474 breast cancer cells.

### Step 1:

Breast cancer cells BT474 were incubated in a 10 cm culture dish with 10 mL of cell culture medium (Iscove's Modified Dulbecco's Medium (IMDM)/ 10% Fetal Bovine Serum (FBS)/ 30ng/mL Epidermal Growth Factor (EGF)) at 37°C, 5% CO₂ for three days. The cell medium was removed and the BT474 cells were washed with 10 mL PBS, then 1 mL of CTS^{™} TrypLE^{™} Select enzyme was added and reacted at 37°C, 5% CO₂ for 5 minutes. Then, 10 mL of cell culture medium was added to collect the cells, which were transferred to a 15 mL centrifuge tube, and after being centrifuged at 400g for 5 minutes at 4-8°C, the supernatant was removed, then the BT474 cells were washed twice with PBS, and finally, the BT474 cells were resuspended in PBS and the cell concentration was adjusted to 5×10⁶-1×10⁷ cells/mL.

### Step 2:

20 µL of DMSO solvent was added to a Deep Red reagent tube, 5 µL of Deep Red solution was taken and added to 5 mL of PBS to prepare a 1 µM CellTracker^{™} Deep Red dye (Invitroge^{™}, C34565) solution, which was stored and protected from light. 2 mL of breast cancer cells BT474 were mixed with 2 mL of 1 µM Deep Red solution at a volume ratio of 1:1, for a total of 4 mL, and then reacted at room temperature, protected from light, for 10 minutes.

### Step 3:

10 mL of cell culture medium (IMDM/10% FBS/30 ng/mL EGF) was taken, and the Deep Red-stained BT474 cells were centrifuged and washed twice at 400g, 4-8°C for 5 minutes. The concentration of the Deep Red-stained breast cancer cells BT474 was adjusted to 1 × 10⁵ cells/mL using the cell culture medium.

### Step 4:

The NK cells in the experimental group, control group, and comparison group from Example 6 were taken and mixed with the CellTracker^{™} Deep Red-stained BT474 cells, and after mixed at a ratio of 1:1, 5:1 and 10:1 to a final volume of 200 µL, placed in a 96-well plate flat bottom, and then reacted at 37°C for 4 hours.

### Step 5:

100 µL of Annexin V/PI solution (by mixing 98.5 µL of 1X binding buffer with 1 µL of Annexin V and 0.5 µL of PI) was prepared. 200 µL of the cytotoxic effect cells after 4 hours of reaction were taken and mixed with 100 µL of Annexin V/PI solution, reacted at room temperature, protected from light, for 5 minutes, and the Deep Red Annexin V⁺/PI⁺ cell population was analyzed by flow cytometry (FACS).

### Experimental Results:

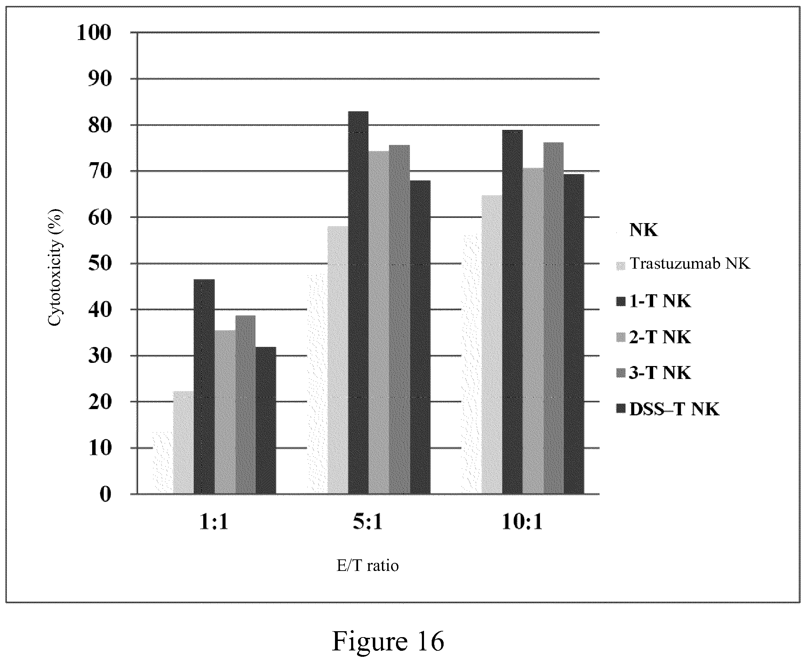

First, the Deep Red⁺ BT474 cell population was selected, then the cell proportion of Annexin V⁺/PI⁺ BT474 cells was analyzed, subtracting the value of spontaneous cell death (Annexin V⁺/PI⁺) in BT474 cells to compare the NK cytotoxicity (specific lysis) effect. Figure 16 shows the effect of different hydrocarbon chain linker conjugated Trastuzumab-NK cells on the cytotoxicity against solid tumor breast cancer cells. From Figure 16, it can be observed that, compared to NK in the control group and Trastuzumab NK and DSS-T NK in the comparison group, the linker-antibody conjugated NK cells in the experimental groups exhibited better cytotoxic effect, in which the experimental group 1-T NK cells demonstrating the best cytotoxicity, with E/T ratios (effector-to-target ratio) of 46.5%, 82.9%, and 78.9% at 1:1, 5:1, and 10:1 ratios, respectively, and as the proportion of NK cells increased, the cytotoxicity effect also improved. The experimental results showed that the 1-T NK cells were the most effective in cytotoxicity among the four types of linker-antibody conjugated NK cells. From Examples 6, 7, and 8, it can be observed that among the four types of linkers, the antibody prepared with linker formula α had the best conjugating ability to the cells and exhibited better cellular efficacy after conjugating.

### Example 9: [Analysis of Cell Apoptosis Caused by Different Hydrocarbon Chain Linker Conjugated Trastuzumab-NK Cells against Three Types of Solid Tumor Cancer Cells]

The following describes the steps for comparing the cell apoptosis caused by different hydrocarbon chain linker conjugated Trastuzumab-NK cells against three types of solid tumor cancer cells.

### Step 1:

1 mL of Frozen Human NK cells (TCI GENE; Lot.no. 20220627) and 10 mL of PBS were added to a 15mL centrifuge tube, and centrifuged and washed at 400g for 5 minutes. The NK cell concentration was adjusted to 5 × 10⁵ cells/mL using NK culture medium (RPMI, 5% EliteGro, 500 IU/mL rhIL-2), and the cells were transferred to a T75 culture flask for incubation. After incubation at 37°C, 5% CO₂ for one day, the buffer was replaced using one of the following methods (A) or (B):
(A) The NK cells were transferred to a 15 mL centrifuge tube, centrifuged at 400g for 5 minutes at 4-8°C, the supernatant was removed, then the NK cells were washed with 10 mL PBS twice, and the NK cells were resuspended in PBS and the NK cell concentration was adjusted to 2×10⁶ cells/mL using PBS.
(B) The NK cells were transferred to a 50 mL centrifuge tube, centrifuged at 400g for 5 minutes at 4-8°C, the supernatant was removed, then the NK cells were washed with 50 mL PBS once, and the NK cells were resuspended in PBS and the NK cell concentration was adjusted to 2×10⁶ cells/mL using PBS.

Finally, 1 mL of the resulting solution was distributed into each of six tubes and labeled as control group, comparison group, and experimental groups (1), (2), (3), and (4), respectively.

### Step 2:

### [Preparation of Control Group, Comparison Group, and Experimental Groups]

The control group was a mixture formed by adding 1 mL of PBS and 1 mL of NK cell solution; the comparison group was prepared by taking 940 µL of PBS and adding 60 µL of 1mg/ml Trastuzumab solution and DSS-T solution, respectively; the experimental groups were prepared by taking 940 µL of PBS and adding 60 µL of 1mg/mL of 1-T, 2-T, 3-T, and 4-T solutions, respectively (1-T, 2-T, and 3-T are hydrocarbon chain linker conjugated Trastuzumab, and 4-T is formula δ conjugated Trastuzumab). Then the resulting solution was mixed with 1 mL of NK cell solution from Step 1 at a volume of 1:1, resulting in a final Trastuzumab concentration of 30 µg/mL and a final NK cell concentration of 1×10⁶ cells/mL. Finally, the mixtures of the control group, comparison group, and experimental groups were irradiated with UV at a wavelength of 365 nm with 400mJ/cm² and allowed to stand at 2-8°C for 10 minutes.

### Step 3:

The mixtures from Step 2 were divided into two groups for subsequent experiments:
(A) For small-scale cell test: The mixtures from Step 2 were transferred to a 15 mL centrifuge tube and an equal volume of PBS/1%HSA solution was added. After the resulting solution centrifuged at 400g for 5 minutes at 4-8°C, the supernatant was removed, and the NK cells were washed with 10mL PBS/1%HSA twice. The NK cells were resuspended in the solution required for the experiment, and NK cell concentration was adjusted to 2×10⁶ cells/mL.
(B) For large-scale animal test: The mixtures from Step 2 were transferred to a 50 mL centrifuge tube and an equal volume of PBS/1%HSA solution was added, after being centrifuged at 400g for 5 minutes at 4-8°C, the supernatant was removed, then the NK cells were washed with 50 mL PBS/1% HSA once, and the NK cells were resuspended in the solution required for the experiment and the NK cell concentration was adjusted to 2×10⁶ cells/mL.

### Step 4:

### [Conjugation Analysis]

After 200 µL from solutions of each group in Step 2 was taken and transferred into a 1.5 mL test tube (Eppendorf), and Goat F(ab')2 Anti-Human IgG-(Fab')2 (PE) (abcam, cat.no. ab98606) was diluted with PBS at a volume ratio of 1:100, 200 µL of the diluted solution was added to solutions of each group from Step 3 and reacted at 4°C for 15 minutes. 800 µL of PBS was added, the mixture was centrifuged at 400g for 5 minutes at 4-8°C, and the supernatant was removed, then after the cells were washed and centrifuged twice with 1 mL PBS, the supernatant was removed, and the cells were resuspended in 200 µL of PBS and analyzed by flow cytometry (FACS).

### Step 5:

### [Cancer Cell Fluorescence Labeling]

Breast cancer cells BT474, human colorectal cancer cells Colo205, and human pancreatic cancer cells BxPC-3 were incubated in 10 cm culture dishes with 10 mL of cell culture medium (IMDM/10%FBS/30ng/mL EGF) (RPMI/10%FBS) at 37°C, 5% CO₂ for three days. The cell medium was removed and the cells were washed with 10 mL PBS, and 1 mL of CTS^{™} TrypLE^{™} Select enzyme was added and reacted at 37°C, 5% CO₂ for 5 minutes. After adding 10 mL of cell culture medium to collect the cells, the cells were centrifuged at 400g for 5 minutes at 4-8°C, and the supernatant was removed, then the cells were washed twice with PBS, and finally resuspended in PBS and the cell concentration was adjusted to 5×10⁶-1×10⁷ cells/mL.20 µL of DMSO solvent was added to a Deep Red reagent tube, then 5 µL of Deep Red solution was taken and added to 5 mL of PBS to prepare a 1 µM CellTracker^{™} Deep Red dye (Invitrogen^{™}, C34565) solution, which was stored and protected from light. 2 mL of cancer cell suspension was mixed with 2 mL of 1 µM Deep Red solution at a volume ratio of 1:1 for a total of 4 mL, and reacted at room temperature, protected from light, for 10 minutes. 10 mL of cancer cell culture medium was taken and used to centrifuge and wash the Deep Red-stained cancer cells twice under the following condition: 400g, 4-8°C, and 5 minutes. The concentration of Deep Red-stained cancer cells was adjusted to 1×10⁵ cells/mL using the cell culture medium.

### Step 6:

### [Cell apoptosis analysis]

The NK cells from the experimental groups, control group, and comparison group were mixed with the CellTracker^{™} Deep Red dye-stained cancer cells at a ratio of 5:1 in a 96-well flat-bottom plate to a final volume of 200 µL and incubated at 37°C for 4 hours. 100 µL of Annexin V/PI solution was prepared (by mixing 98.5 µL of 1X binding buffer with 1 µL of Annexin V and 0.5 µL of PI). 200 µL of the cytotoxic effect cells after 4 hours of reaction were taken and mixed with 100 µL Annexin V/PI solution, and after reacted at room temperature, protected from light, for 5 minutes, the Deep Red⁺/Annexin V-/PI- cell population was analyzed by flow cytometry (FACS).

### Experimental Results:

Goat F(ab')2 Anti-Human IgG-(Fab')2 (PE) was used for antibody staining on the NK cell membrane, followed by flow cytometry (FACS) analysis of the PE signal to determine the strength of Trastuzumab conjugation to NK cells.

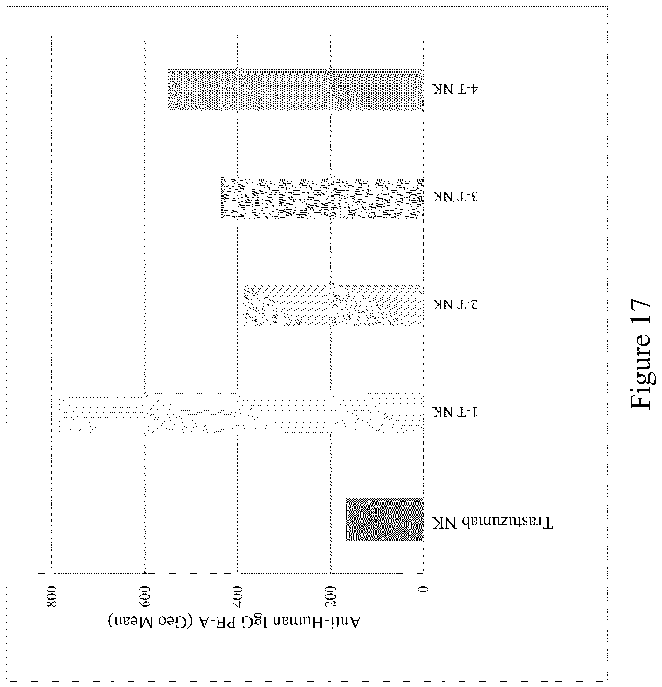

Figure 17 shows the results of the conjugating degree between NK cells and different linker conjugated Trastuzumab. From Figure 17, it can be observed that, compared to the control group Trastuzumab NK, the experimental groups 1-T NK, 2-T NK, 3-T NK, and 4-T NK were successfully conjugated to the surface of NK cells.

Then, the Deep Red⁺ cancer cell population was selected, and the cell proportion of NK cells-induced cancer cell apoptosis was analyzed (100%-viable cancer cells% (Annexin V-/PI-)), then the spontaneously apoptotic cancer cells were subtracted to compare the apoptosis results caused by different chain linker conjugated Trastuzumab-NK cells against three types of solid tumors (breast cancer, colorectal cancer, and pancreatic cancer cells).

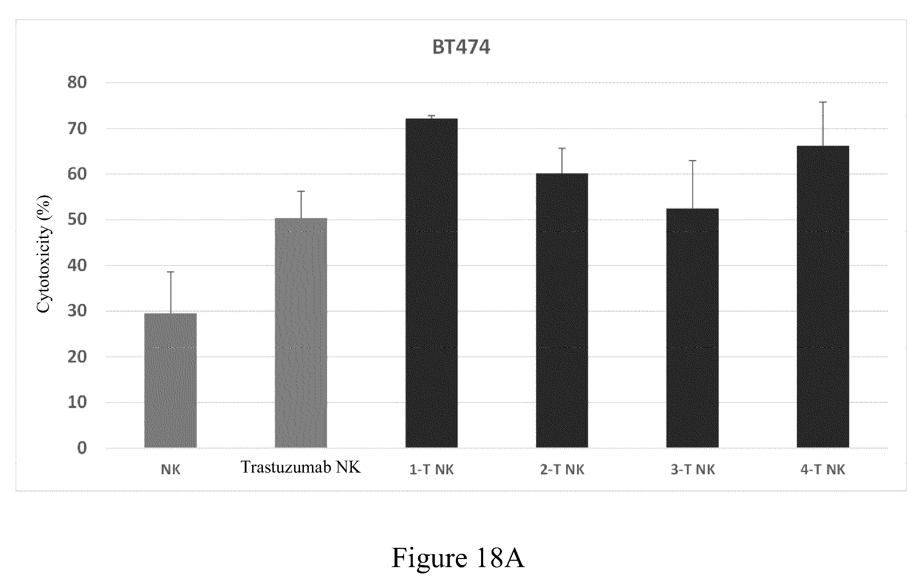

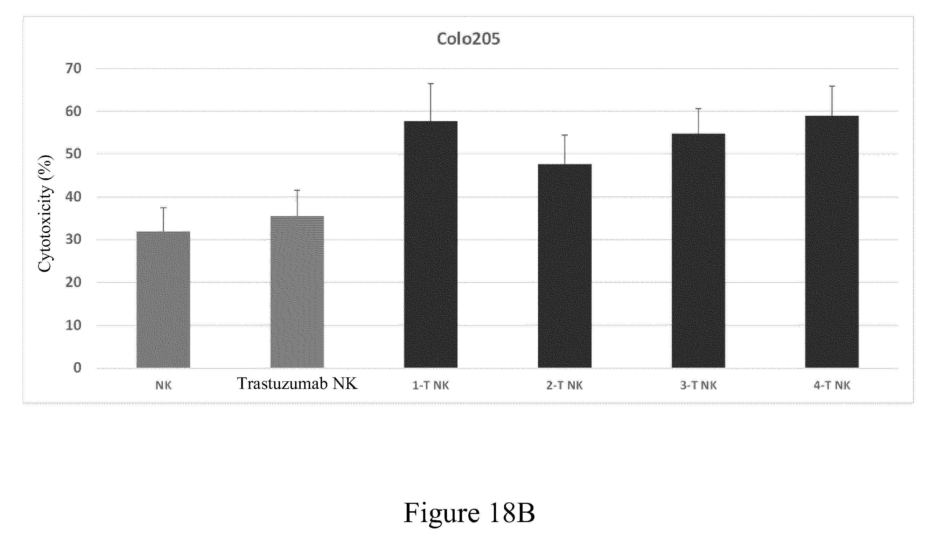

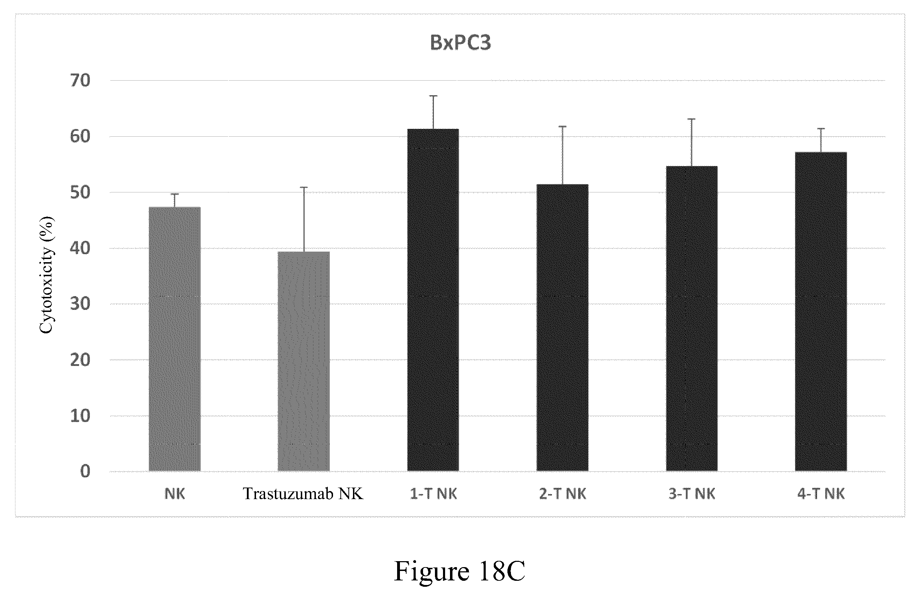

Figures 18A-18C show the cancer cell apoptosis results of three types of solid tumor cancer cells caused by different linker conjugated Trastuzumab NK cells. From Figures 18A-18C, it can be observed that, compared to the NK cells in the control group and the Herceptin+NK in the comparison group, the four types of linker-antibody conjugated NK cells against three types of solid tumors (including breast cancer (BT474), colorectal cancer (Colo205), and pancreatic cancer cells (BxPC3)) exhibited better apoptosis effects, in which the experimental groups 1-T NK and 4-T NK cells demonstrated better apoptosis capabilities, with apoptosis rates of 72.2%/66.2%, 57.7%/58.9%, and 61.4%/57.1% in breast, colorectal, and pancreatic cancer cells, respectively, which showed that the 4-T linker conjugated Trastuzumab-NK cells, having the PEG structure, exhibited similar apoptosis-inducing effects on solid tumor cells as the hydrocarbon chain linker conjugated Trastuzumab-NK, compared to hydrocarbon chain linker conjugated Trastuzumab. Furthermore, the experiment demonstrated that the cytotoxic strategy of using linker conjugated Trastuzumab-NK cells for solid killing tumor cells with cytotoxicity can be extended beyond breast cancer to other types of solid tumor cancers.

### Example 10 [Comparative Analysis of the Reaction Time Required for Linker Conjugated Trastuzumab to Conjugate to NK Cells]

The following describes the steps for comparing the reaction time required for linker conjugated Trastuzumab to conjugate to NK cells.

### Step 1:

1 mL of Frozen Human NK cells (TCI GENE; Lot.no. 20210816) and 10 mL of PBS were transferred to a 15 mL centrifuge tube, centrifuged and washed at 400g for 5 minutes, then transferred to a 10 cm cell culture dish with 10 mL of NK culture medium (RPMI, 5% EG, 500 IU/mL rhIL-2) for incubation. After incubating at 37°C, 5% CO₂ for one day, the NK cells were washed and the buffer was replaced using the following methods (A) or (B):
(A) The NK cells were transferred to a 15 mL centrifuge tube, centrifuged at 400g at 4-8°C for 5 minutes, the supernatant was removed, then the NK cells were washed twice with 10 mL PBS, and finally NK cells were resuspended in PBS and the NK cell concentration was adjusted to 2×10⁶ cells/mL using PBS.
(B) The NK cells were transferred to a 50 mL centrifuge tube, centrifuged at 400g at 4-8°C for 5 minutes, the supernatant was removed, then the NK cells were washed once with 50 mL PBS, and finally NK cells were resuspended in PBS and the NK cell concentration was adjusted to 2×10⁶ cells/mL using PBS.

Finally, 1 mL of the resulting solution was distributed into each of the 5 tubes and labeled as control group, comparison group, and experimental groups (1), (2), and (3), respectively.

### Step 2:

### [Preparation of Control Group, Comparison Group, and Experimental Groups]

The control group was a mixture formed by adding 1 mL of PBS and 1 mL of NK cell solution; the comparison group was prepared by taking 900 µL of PBS and adding 100 µL of 1 mg/mL Trastuzumab solution; the experimental groups were prepared by taking 900 µL of PBS and adding 100 µL of 1 mg/mL linker conjugated Trastuzumab solution. Then, the resulting solution was mixed with 1 mL of NK cell solution from Step 1 at a volume of 1:1 to make the final Trastuzumab concentration of 50 µg/mL and the NK cell concentration of 1×10⁶ cells/mL. Then the solutions were then uniformly irradiated with UV at a wavelength of 365 nm with 400 mJ/cm², and the mixtures of the control group, comparison group, and experimental groups were allowed to stand at 2-8°C for 10 minutes. Finally, after the mixtures of the control group, comparison group, and experimental groups were allowed to stand at 4°C for 1, 5, or 10 minutes, respectively, a quenching solution (RPMI/10% FBS) was added to terminate the conjugation reaction between the linker conjugated Trastuzumab and the cells.

### Step 3:

The mixtures from Step 2 were divided into the following two groups for subsequent experiments:
(A) For small-scale cell test: The mixtures from Step 2 were transferred to a 15 mL centrifuge tube and an equal volume of PBS/1%HSA solution was added. After the resulting solutions were centrifuged at 400g for 5 minutes at 4-8°C, the supernatant was removed, then the NK cells were washed with 10 mL PBS/1%HSA twice, and the NK cells were resuspended in the solution required for the experiment and the NK cell concentration was adjusted to 2×10⁶ cells/mL.
(B) For large-scale animal test: The mixtures from Step 2 were transferred to a 50 mL centrifuge tube and an equal volume of PBS/1%HSA solution was added. After the resulting solutions were centrifuged at 400g for 5 minutes at 4-8°C, the supernatant was removed, then the NK cells were washed with 50 mL PBS/1%HSA once, and the NK cells were resuspended in the solution required for the experiment and NK cell concentration was adjusted to 2×10⁶ cells/mL.

### Step 4:

After 200 µL from solutions of each group in Step 3 was taken and transferred into a 1.5 mL test tube (Eppendorf), and Goat F(ab')2 Anti-Human IgG-(Fab')2 (PE) (abcam, cat.no. ab98606) was diluted in PBS at a volume ratio of 1:100, reacted at 4°C for 30 minutes. 800 µL of PBS was added, After the mixture was centrifuged at 400g for 5 minutes at 4-8°C, the supernatant was removed, then after the cells were washed twice with 1 mL PBS, the supernatant was removed, and 200 µL of PBS was added to each group to resuspend the cells and analyzed using flow cytometry (FACS).

### Experimental Results:

Goat F(ab')2 Anti-Human IgG-(Fab')2 (PE) was used to carry out antibody staining on the NK cell membrane, followed by flow cytometry (FACS) analysis of the PE signal strength to obtain the strength of Trastuzumab conjugating to NK cells.

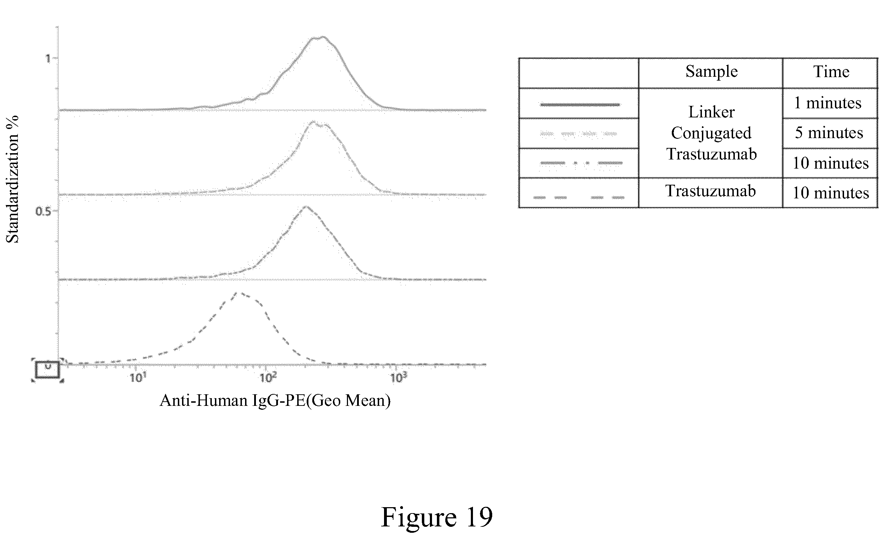

Figure 19 shows the results of linker conjugated Trastuzumab to NK cells at different conjugating reaction times. From Figure 19, it can be observed that, compared to the control group Trastuzumab, linker conjugated Trastuzumab in all experimental groups at 1, 5, and 10 minutes successfully conjugated to the surface of NK cells.

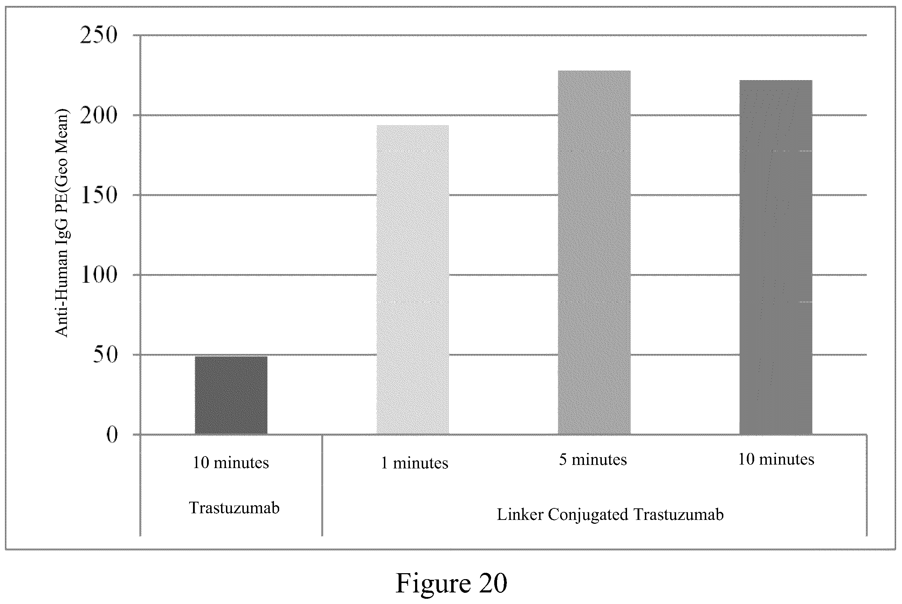

Figure 20 shows the conjugating degree of linker conjugated Trastuzumab to the surface of NK cells at different times. From Figure 20, it can be observed that the geometric mean values (Geo Mean) of the experimental groups at 1, 5, and 10 minutes were 194, 228, and 222, respectively. The differences among these values were minor, representing that linker conjugated Trastuzumab could efficiently conjugate to the surface of NK cells within just 1 minute.

### Example 11 [Analysis of the Conjugating Ability of Linker Conjugated Trastuzumab against Different Cells: Platelets]

The following describes the steps for analyzing the conjugating ability of linker conjugated Trastuzumab to different cells: platelet.

### Step 1:

After 15 mL of fresh blood was mixed with PBS at a ratio of 1:1, 15 mL of Ficoll-Paque Plus/Premium was added to a 50c.c. centrifuge tube, then the blood and PBS mixture was slowly added, and centrifuged at 600g for 20 minutes and the deceleration set was turned to 0. After centrifugation, the upper plasma layer was removed, and the peripheral blood mononuclear cell (PBMC) layer was transferred to a new 50c.c. centrifuge tube, PBS was added to a total volume of 40mL and centrifuged at 400g for 5 minutes. The supernatant (containing the platelets) was transferred to a new 50c.c. centrifuge tube, PBS was added to a total volume of 40mL, and centrifuged at 800g for 10 minutes. The supernatant was removed, PBS was added to a total volume of 40mL, and centrifuged at 800g for 10 minutes. Finally, the supernatant was removed, the cells were resuspended in PBS, and the cell concentration was adjusted to 1×10⁷ cells/mL for subsequent experiments.

### Step 2:

### [Preparation of Control Group, Comparison Group, and Experimental Groups]

The control group was a mixture formed by adding 1 mL of PBS and 1 mL of platelet cell solution; the comparison group was prepared by taking 900 µL of PBS and adding 100 µL of 1 mg/mL Trastuzumab solution; the experimental group was prepared by taking 900 µL of PBS and adding 100 µL of 1 mg/mL linker formula α conjugated Trastuzumab. Each solution was irradiated uniformly with UV light at a wavelength of 365 nm for 10 seconds, followed by a standing period of 10 seconds each time, repeated twice in total, then 1 mL of the platelet cell solution from Step 1 was added, resulting in a mixture with a final Trastuzumab antibody concentration of 50 µg/mL and a mixture with a final concentration of different linker conjugated Trastuzumab of 50 µg/mL. Then, the mixtures of control group, comparison group, and experimental group were allowed to stand at 4°C for 10 minutes.

### Step 3:

The mixtures from Step 2 were centrifuged at 800g for 5 minutes at 4-8°C, the supernatants were removed, then the cells were washed twice with 10 mL PBS, and finally the cells were then resuspended in cell culture medium (RPMI/10% FBS) and adjusted to a concentration of 1×10⁶ cells/mL for subsequent experiments.

### Step 4:

200 µL from solutions of each group in Step 3 was taken and transferred into a 1.5 mL test tube (Eppendorf). After Goat F(ab')2 Anti-Human IgG-(Fab')2 (PE) (abcam, cat.no. ab98606) was diluted in PBS at a volume ratio of 1:100, 200 µL of this diluted solution was added to the solutions of each group, along with 5 µL of FITC Anti-Human CD41 antibody (BioLegend, 303704), and the mixtures were incubated at 4°C for 30 minutes. After 800 µL of PBS was added and centrifuged at 800g for 5 minutes at 4-8°C, the supernatant was removed, and then the cells were washed twice with 1 mL PBS. Then, the supernatant was removed, 200 µL of PBS was added to resuspend the cells, and finally analyzed by flow cytometry (FACS) analysis.

### Experimental Results:

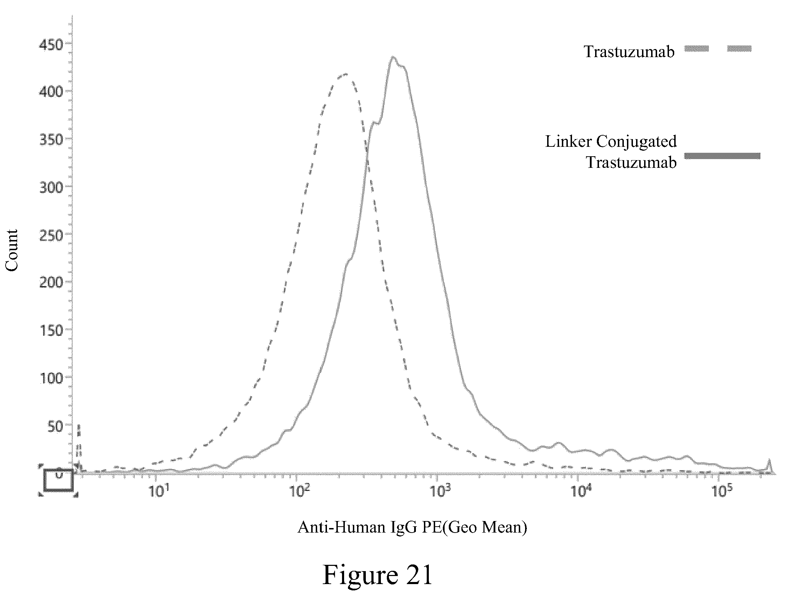

Goat F(ab')2 Anti-Human IgG-(Fab')2 (PE) was used to carry out antibody staining on the CD41⁺ platelet cell membrane, followed by flow cytometry (FACS) analysis of the PE signal strength to obtain the degree of antibody conjugating to platelet cells. Figure 21 shows the results of the conjugating response of linker formula α conjugated Trastuzumab to platelets. It can be observed that the experimental group were successfully conjugated to the surface of CD41⁺ platelets.

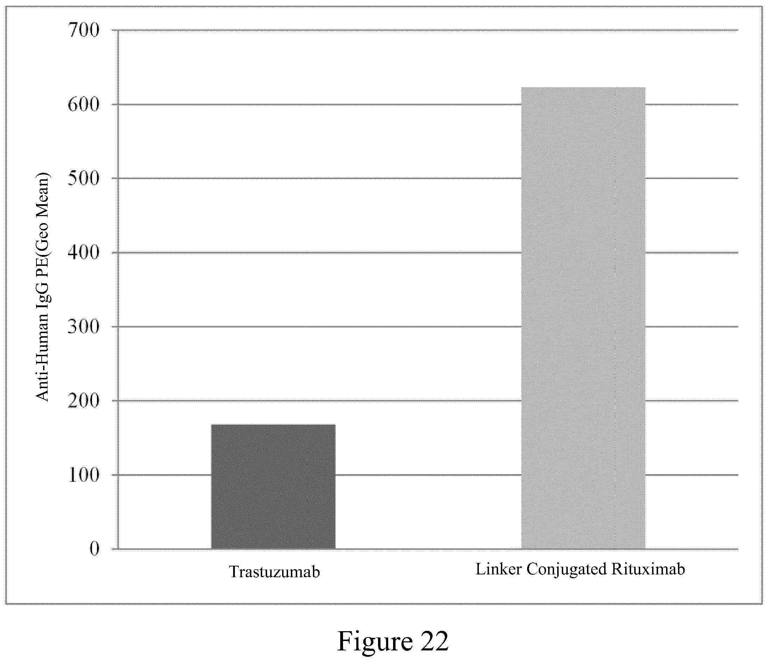

Figure 22 shows the conjugating ability of linker formula α conjugated Trastuzumab to CD41⁺ platelets. From Figure 22, it can be observed that, compared to the Trastuzumab in comparison group, the experimental group exhibited greater conjugation efficiency to CD41⁺ platelets.

### Example 12 [Analysis of the Conjugating Ability of Linker Conjugated Trastuzumab to Different Cells: CD3+/CD8+ T Cells]

The following describes the steps for analyzing the conjugating ability of linker conjugated Trastuzumab to different cells: CD3⁺/CD8⁺ T Cells

### Step 1:

15mL of fresh blood was mixed with PBS at a ratio of 1:1, then 15mL of Ficoll-Paque Plus/Premium was added to a 50 c.c. centrifuge tube, then the blood and PBS mixture was slowly added, and centrifuged at 600g for 20 minutes and the deceleration set was turned to 0. After centrifugation, the upper plasma layer was removed, then the PBMC layer was taken and transferred to a new 50 c.c. centrifuge tube, PBS was supplemented to make a total volume of 40 mL, centrifuged at 400g for 5 minutes, and the supernatant was removed. PBS was added to make a total volume of 40 mL, after being centrifuged at 400g for 5 minutes, the supernatant was removed, and finally PBS was added and the cell concentration was adjusted to 2×10⁶ cells/mL for subsequent experiments.

### Step 2:

### [Preparation of Control Group, Comparison Group, and Experimental Group]

The control group was a mixture formed by adding 1 mL of PBS and 1 mL of PBMC cell solution; the comparison group was prepared by taking 900 µL of PBS and adding 100 µL of 1 mg/mL Herceptin solution; the experimental group was prepared by taking 900 µL of PBS and adding 100 µL of 1 mg/mL of linker conjugated Trastuzumab solution. The solutions were uniformly irradiated with UV at a wavelength of 365 nm for 10 seconds, followed by a standing period of 10 seconds each time, repeated twice in total, and 1 mL of the PBMC solution from Step 1 was added, resulting in a mixture with a final Trastuzumab antibody concentration of 50 µg/mL and a mixture with a final linker conjugated Trastuzumab concentration of 50 µg/mL. Then the mixtures of the control group, comparison group, and experimental group were allowed to stand at 4°C for 10 minutes.

### Step 3:

After the mixtures from Step 2 were centrifuged at 400g for 5 minutes at 4-8°C, the supernatant was removed, the cells were washed twice with 10 mL PBS, and finally the cells were resuspended in cell culture medium (RPMI/10%/FBS) and the cell concentration was adjusted to 1×10⁶ cells/mL for subsequent experiments.

### Step 4:

After 200 µL from solutions of each group in Step 3 was taken and transferred into a 1.5 mL test tube (Eppendorf) and Goat F(ab')2 Anti-Human IgG-(Fab')2 (PE) (abcam, cat.no. ab98606) was diluted in PBS at a volume ratio of 1:100, 200 µL of the diluted solution was added to solutions of each group. 5 µL of FITC Anti-Human CD3 antibody (BioLegend, 317306) and 5 µL of PerCP/Cyanine5.5 Anti-Human CD8 antibody (BioLegend, 344710) were added to each group simultaneously, and then placed and reacted at 4°C for 30 minutes. Then, 800 µL of PBS was added, after being centrifuged at 400g for 5 minutes at 4-8°C, the supernatant was removed, then the cells were washed twice with 1 mL PBS, and the supernatant from each group was removed and 200 µL of PBS was added to resuspend the cells, and finally analyzed by flow cytometry (FACS).

### Experimental Results:

Goat F(ab')2 Anti-Human IgG-(Fab')2 (PE) was used to carry out antibody staining on the CD3⁺/CD8⁺ T cell membrane, followed by flow cytometry (FACS) analysis of the PE signal to obtain the degree of antibody conjugating to PBMC cells.

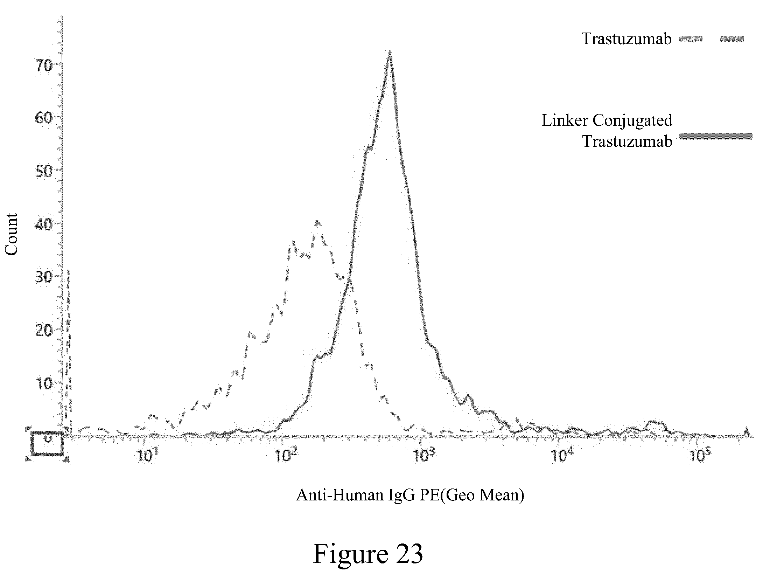

Figure 23 shows the results of the conjugating response of linker (formula α) conjugated Trastuzumab to CD3⁺/CD8⁺ T cells. From Figure 23, it can be observed that the experimental group with linker (formula α) conjugated Trastuzumab were successfully conjugated to the surface of CD3⁺/CD8⁺ T cells.

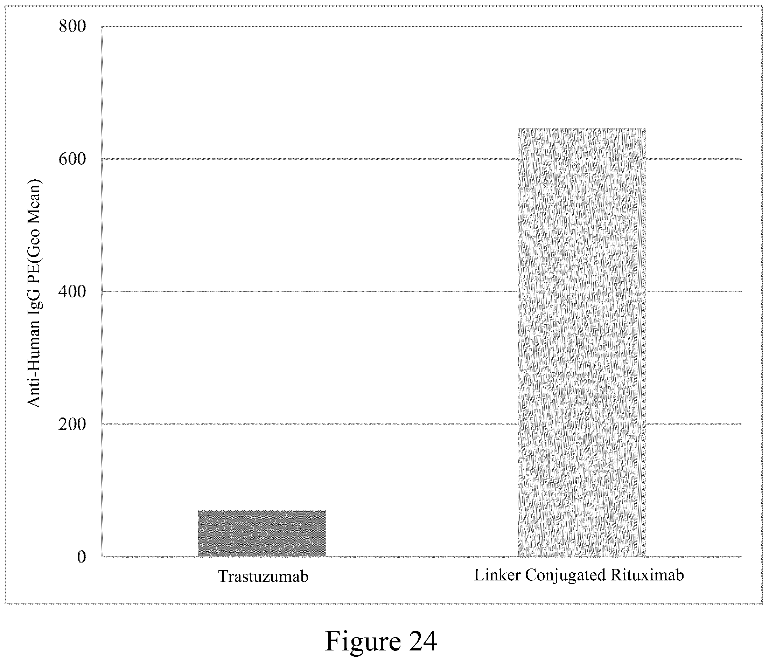

Figure 24 shows the conjugating ability of linker (formula α) conjugated Trastuzumab to CD3⁺/CD8⁺ T cells. From Figure 24, it can be observed that, compared to the Trastuzumab in the comparison group, the experimental group with linker (formula α) conjugated Trastuzumab had a better conjugating ability to CD3⁺/CD8⁺ T cells.

### Example 13 [Cellular Stability Test of Linker Conjugated Trastuzumab]

The following describes the steps for the cellular stability test of linker conjugated Trastuzumab.

### Step 1:

15 mL of fresh blood was mixed with PBS at a ratio of 1:1, 15 mL of Ficoll-Paque Plus/Premium was added to a 50c.c. centrifuge tube, then the blood and PBS mixture was slowly added, and centrifuged at 600g for 20 minutes and the deceleration set was turned to 0. After centrifugation, the upper plasma layer was removed and the PBMC layer was transferred to a new 50 c.c. centrifuge tube, PBS was supplemented to make a total volume of 40 mL, and centrifuged at 400g for 5 minutes. The supernatant was removed, PBS was added to make a total volume of 40 mL, centrifuged at 400g for 5 minutes, and the supernatant was removed. Finally, PBS was added and the cell concentration was adjusted to 2×10⁶ cells/mL for subsequent experiments.

### Step 2:

### [Preparation of Control Group, Comparison Group, and Experimental Group]

The control group was a mixture formed by adding 1 mL of PBS and 1 mL of PBMC cell solution; the comparison group was prepared by taking 900 µL of PBS and adding 100 µL of 1 mg/mL Herceptin solution; the experimental group was prepared by taking 900 µL of PBS and adding 100 µL of 1 mg/mL of linker conjugated Trastuzumab solution. The solutions were uniformly irradiated with UV at a wavelength of 365 nm for 10 seconds, followed by a standing period of 10 seconds each time, repeated twice in total. Then, 1 mL of the PBMC solution from Step 1 was added, resulting in a mixture with final Trastuzumab antibody concentration of 50 µg/mL and a mixture with final linker conjugated Trastuzumab concentration of 50 µg/mL of. Then, the mixtures of the control group, comparison group, and experimental group were allowed to stand at 4°C for 10 minutes.

### Step 3:

After the mixtures from Step 2 were centrifuged at 400g for 5 minutes at 4-8°C, the supernatant was removed, the cells were washed and centrifuged twice with 10 mL PBS. Finally, the cells were resuspended in cell culture medium (RPMI/10% FBS), and the cell concentration was adjusted to 1×10⁶ cells/mL for subsequent experiments.

### Step 4:

The linker conjugated Trastuzumab-PBMC cells, Trastuzumab-PBMC cells, and PBMC cells were seeded to 96-well flat-bottom plate at a final volume of 200 µL/well. The cells were then placed in a 37°C cell culture incubator with six replicate wells per group, and supplemented with 50 µL of cell culture medium every 24 hours.

### Step 5:

Every 24 hours, after 200 µL of the solution from each group in Step 4 was transferred into a 1.5 mL test tube (Eppendorf) and Goat F(ab')2 Anti-Human IgG-(Fab')2 (PE) (abcam, cat.no. ab98606) was diluted in PBS at a volume ratio of 1:100, 200 µL of the diluted solution was taken and added to solutions of each group and 5 µL of FITC Anti-Human CD16 monoclonal antibody (eBio-science^{™}, 17-0168-42) was added to each group at the same time, and reacted at 4°C for 30 minutes.800 µL of PBS was added, after being centrifuged at 400g for 5 minutes at 4-8°C, the supernatant was removed, then the cells were washed twice with 1 mL PBS, the supernatant was removed, and 200µL of PBS was added to resuspend the cells, and finally analysis was performed using flow cytometry (FACS). Step 5 was repeated every 24 hours until 96 hours.

### Experimental Results:

Goat F(ab')2 Anti-Human IgG-(Fab')2 (PE) was used to carry out antibody staining on the CD16⁺NK cell membrane, followed by flow cytometry (FACS) analysis of the PE signal to obtain the degree of linker conjugated Trastuzumab conjugating to CD16⁺ NK cells at various time points.

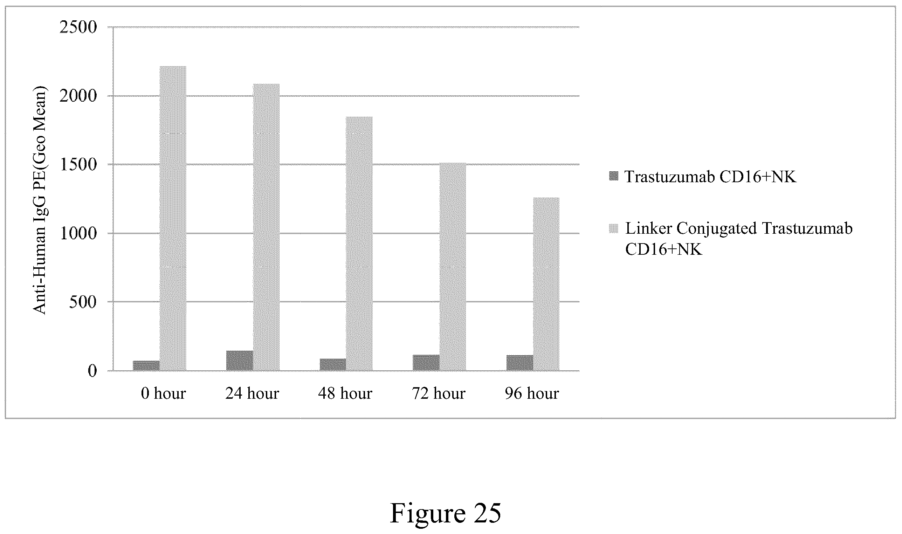

Figure 25 shows the conjugating degree of linker conjugated Trastuzumab-CD16⁺ NK cell membrane at different times. From Figure 25, it can be observed that the arithmetic mean signal of antibody staining at 0 hours was 2219, and after incubation in a 37°C cell culture incubator for 96 hours, the arithmetic mean signal decreased by about 45% to 1261. From this, it can be observed that the half-life of the linker conjugated Trastuzumab conjugated on the surface of CD16⁺ NK cells was about 4 days.

### Example 14: [Viability Test of Linker Conjugated Trastuzumab Cells]

The following describes the steps for the viability test of linker conjugated Trastuzumab.

### Step 1:

After 15 mL of fresh blood was mixed with PBS at a ratio of 1:1, 15 mL of Ficoll-Paque Plus/Premium was added to a 50 c.c. centrifuge tube, then the blood and PBS mixture was slowly added, and centrifuged at 600g for 20 minutes and the deceleration set was turned to 0. After centrifugation, the upper plasma layer was removed and the PBMC layer was transferred to a new 50 c.c. centrifuge tube, and PBS was supplemented to make a total volume of 40 mL. Then, after being centrifuged at 400g for 5 minutes, the supernatant was removed, and then PBS was added to make a total volume of 40 mL. Then, after being centrifuged at 400g for 5 minutes, the supernatant was removed, and finally PBS was added and the cell concentration was adjusted to 2×10⁶ cells/mL for subsequent experiments.

### Step 2:

### [Preparation of Comparison Group and Experimental Group]

The comparison group was a mixture formed by adding 1 mL of PBS and 1 mL of PBMC cell solution; the experimental groups were prepared by taking 900 µL of PBS and adding 100 µL of 1 mg/mL of linker conjugated Trastuzumab solution. The solutions were uniformly irradiated with UV at a wavelength of 365 nm for 10 seconds, followed by a standing period of 10 seconds each time, repeated twice in total. Then, 1 mL of the PBMC cell solution from Step 1 was added, resulting in a final concentration of 50 µg/mL of linker conjugated Trastuzumab in the mixture. The mixtures of the comparison group and experimental group were allowed to stand at 4°C for 10 minutes.

### Step 3:

The mixtures from Step 2 were centrifuged at 400g for 5 minutes at 4-8°C, the supernatant was removed, then the cells were washed twice with 10 mL PBS, and finally the cells were resuspended in cell culture medium (RPMI/10% FBS) and the cell concentration was adjusted to 1×10⁶ cells/mL for subsequent experiments.

### Step 4:

The linker conjugated Trastuzumab-PBMC cells and PBMC cells were seeded to 96-well flat-bottom plate at a final volume of 200 µL/well. The cells were then incubated in a 37°C cell culture incubator with six replicate wells per group, and supplemented with 50 µL of cell culture medium every 24 hours.

### Step 5:

Every 24 hours, 200 µL of the solution from each group in Step 4 was transferred into a 1.5 mL test tube (Eppendorf), 5 µL of anti-CD16 monoclonal antibody (eBioscience^{™}, 17-0168-42) was added to both groups, respectively, and reacted at 4°C for 30 minutes. Then, the cells from both groups were mixed with 100 µL of Annexin V/PI solution/sample, reacted at room temperature, protected from light, for 5 minutes, and the Annexin V-/PI- cell population was analyzed using flow cytometry (FACS).

### Experimental Results:

First, the CD16⁺ NK cell population in the PBMCs was selected, and then the cell proportion of Annexin V/PI-stained cells was used for cell viability analysis.

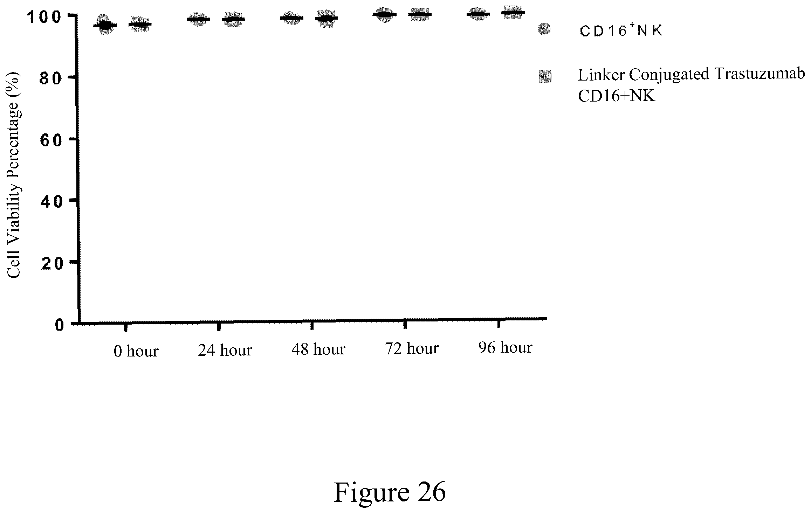

Figure 26 shows the result of the cell viability after 96 hours of conjugating linker conjugated Trastuzumab to CD16⁺ NK cells. From Figure 26, it can be observed that the survival rate of CD16⁺ NK cells conjugated with linker conjugated Trastuzumab all remained above 95%, showing no significant difference compared to the PBMC comparison group. Thus, it can be observed that the conjugation of linker conjugated Trastuzumab to PBMCs did not affect the viability of immune cells such as CD16⁺ NK cells.

### Example 15: [Linker Formula α Enables Rituximab to Conjugate to Cells]

The following describes the steps that linker formula α enables Rituximab to conjugate to cells:

### Step 1:

1mL of a 10 mg/mL commercially available MabThera antibody (Roche, Rituximab) was transferred into an Amicon^{®} Ultra Centrifugal Filter. Then, 13 mL of PBS was added and centrifuged at 2500g for 30 minutes at 4-8°C, the waste liquid from the bottom of the Amicon^{®} Ultra Centrifugal Filter was removed. 8 mL of PBS was added from the top, and centrifuged at 2500g for 30 minutes at 4-8°C. Then, absorbance at 280 nm (OD280) was measured to calculate the concentration, and the antibody concentration was adjusted to 2mg/mL with PBS for subsequent use.

### Step 2:

2.25 mg of the linker formula α from Example 1 was mixed with 590 µL of DMSO solvent to prepare 10mM linker solution.

### Step 3:

3 mL of the Rituximab prepared in Step 1 was mixed with 20.69 µL of the 10 mM linker solution from Step 2, and the mixture was reacted at room temperature for 30 minutes to generate the linker conjugated Rituximab.

### Step 4:

The linker conjugated Rituximab solution was transferred into an Amicon^{®} Ultra Centrifugal Filter, then 11 mL PBS was added, centrifuged at 2500g for 30 minutes at 4-8°C, and the waste liquid from the bottom of the Amicon^{®} Ultra Centrifugal Filter was removed, 6 mL of PBS was added from the top, centrifuged at 2500g for 30 minutes at 4-8°C, and then the waste liquid from the bottom of the Amicon^{®} Ultra Centrifugal Filter was removed. Then, absorbance at 280 nm (OD280) was measured to calculate the concentration, the antibody concentration was adjusted to 5 mg/mL with PBS, mixed with 62.5% glycerol at a volume of 1 (antibody) : 4 (glycerol), and stored at -20°C to obtain a final antibody concentration of 1 mg/mL for subsequent use.

### Step 5:

1 mL of Frozen Human NK cells (TCI GENE; Lot.no. 20211014) and 10 mL of PBS were transferred into a 15 mL centrifuge tube, centrifuged and washed at 400g for 5 minutes, and transferred to a 10cm cell culture dish with 10mL of NK culture medium (RPMI culture medium, 5% EG, 500 IU/mL rhIL-2) for incubation. After incubating at 37°C, 5% CO₂ for one day, the NK cells were washed and the buffer was replaced using the following methods (A) or (B):
(A) The NK cells were transferred to a 15 mL centrifuge tube, centrifuged at 400g at 4-8°C for 5 minutes, the supernatant was removed, then the NK cells were washed twice with 10 mL PBS, and finally NK cells were resuspended in PBS and the NK cell concentration was adjusted to 2×10⁶ cells/mL using PBS.
(B) The NK cells were transferred to a 50 mL centrifuge tube, centrifuged at 400g at 4-8°C for 5 minutes, the supernatant was removed, then the NK cells were washed once with 50 mL PBS, and finally NK cells were resuspended in PBS and the NK cell concentration was adjusted to 2×10⁶ cells/mL using PBS. Finally, 1 mL of the resulting solution was distributed into each of the 3 tubes and labeled as control group, comparison group, and experimental group (1), (2), and (3), respectively.

### Step 6:

### [Preparation of Control Group, Comparison Group, and Experimental Group]

The control group was a mixture formed by adding 1mL of PBS and 1mL of NK cell solution; the comparison group was prepared by taking 900 µL of PBS and adding 100 µL volume of 1mg/mL linker conjugated Rituximab solution, respectively; the experimental group was prepared by taking 900µL of PBS and adding 100µL of 1 mg/mL linker conjugated Rituximab solution, respectively. Then, the resulting solution was mixed with 1 mL of NK cell solution from Step 5 at a volume of 1:1 to make the final Rituximab concentration of 50 µg/mL and the NK cell concentration of 1×10⁶ cells/mL. Then, the solutions were uniformly irradiated with UV at a wavelength of 365 nm with 400 mJ/cm², and the mixtures of the control group, comparison group, and experimental group were allowed to stand at 2-8°C for 10 minutes.

### Step 7:

The mixtures from Step 6 were divided into the following two groups for subsequent experiments:
(A) For small-scale cell test: The mixtures from Step 2 were transferred to a 15 mL centrifuge tube and an equal volume of PBS/1%HSA solution was added. After the resulting solutions were centrifuged at 400g for 5 minutes at 4-8°C, the supernatant was removed, then the NK cells were washed with 10 mL PBS/1%HSA twice, and the NK cells were resuspended in the solution required for the experiment and the NK cell concentration was adjusted to 2×10⁶ cells/mL.
(B) For large-scale animal test: The mixtures from Step 2 were transferred to a 50 mL centrifuge tube and an equal volume of PBS/1%HSA solution was added. After the resulting solutions were centrifuged at 400g for 5 minutes at 4-8°C, the supernatant was removed, then the NK cells were washed with 50 mL PBS/1%HSA once, and the NK cells were resuspended in the solution required for the experiment and the NK cell concentration was adjusted to 2×10⁶ cells/mL.

### Step 8:

After 200 µL of the solution from each group in Step 7 was taken into a 1.5 mL test tube (Eppendorf) tube and Goat F(ab')2 Anti-Human IgG-(Fab')2 (PE) (abcam, cat. no. ab98606) was diluted in PBS at a volume ratio of 1:100, 200 µL of this dilution was added to solutions of each group from Step 6, and reacted at 4°C for 30 minutes. Then, 800 µL of PBS was added, after being centrifuged at 400g for 5 minutes at 4-8°C, the supernatant was removed, then the cells were centrifuged and washed twice with 1 mL PBS, and the supernatant was removed. Finally, the cells were resuspended in 200 µL PBS for each group, and analyzed by flow cytometry (fluorescence activated cell sorting, FACS).

### Experimental Results:

Goat F(ab')2 Anti-Human IgG-(Fab')2 (PE) was used to carry out antibody staining on the NK cell membrane, followed by flow cytometry (FACS) analysis of the PE signal to obtain the strength of antibody conjugating to NK cells.

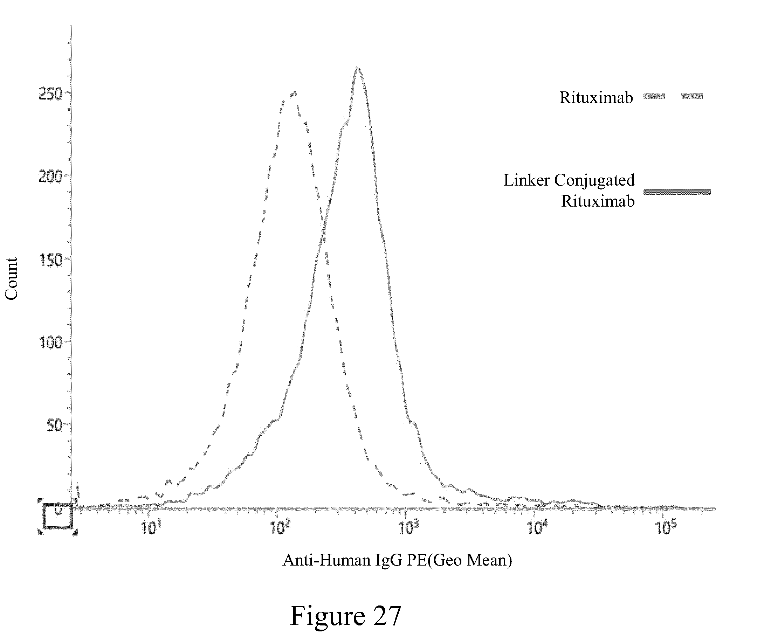

Figure 27 shows the results of the conjugation between linker conjugated Rituximab and NK cells. From Figure 27, it can be observed that the linker conjugated Rituximab in the experimental group was successfully conjugated to the surface of NK cells.

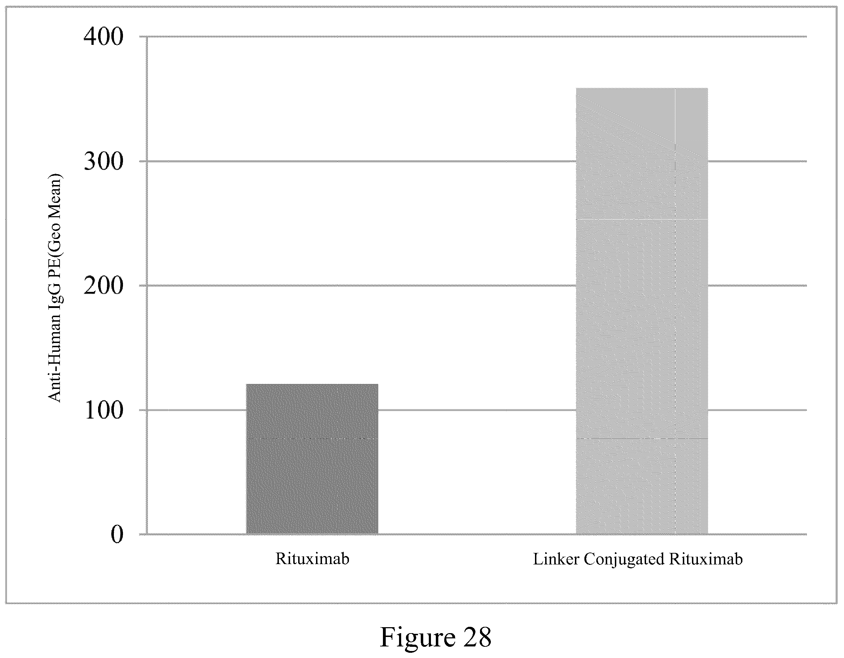

Figure 28 shows the conjugating ability of linker conjugated Rituximab to NK cells. From Figure 28, it can be observed that, compared to Rituximab in the comparison group, the linker (formula α) conjugated Rituximab in the experimental group had a better conjugating ability to NK cells. Thus, the experiment demonstrated that the strategy of using a linker to conjugate antibodies to cells could be extended to antibodies beyond Trastuzumab.

### Example 16 [Linker Formula α Enables Atezolizumab to Conjugate to Cells]

The following describes the steps that linker formula α enables different antibody (Atezolizumab) to bind to cells:

### Step 1:

0.5 mL of 60 mg/mL commercially available antibody Tecentriq (Roche, Atezolizumab) was transferred into an Amicon^{®} Ultra Centrifugal Filter, then 13 mL of PBS was added, centrifuged at 2500g for 30 minutes at 4-8°C, and the waste liquid was removed from the bottom of the Amicon^{®} Ultra Centrifugal Filter. 8 mL of PBS was added from the top, and the mixture was centrifuged at 2500g for 30 minutes at 4-8°C. Then, the absorbance at 280 nm (OD280) was measured to calculate the concentration, and finally the antibody concentration was adjusted to 2mg/mL with PBS for subsequent use.

### Step 2:

2.25 mg of the linker formula α from Example 1 was mixed with 590 µL of DMSO solvent to prepare a 10mM linker solution.

### Step 3:

4 mL of the Atezolizumab solution prepared in Step 1 was taken and mixed with 27.58 µL of the 10 mM linker solution prepared in Step 2, and reacted at room temperature for 30 minutes to generate linker conjugated Atezolizumab.

### Step 4:

The linker conjugated Atezolizumab solution was transferred into an Amicon^{®} Ultra Centrifugal Filter, then 11 mL of PBS was added, after being centrifuged at 2500g for 30 minutes at 4-8°C, the waste liquid was removed from the bottom of the Amicon^{®} Ultra Centrifugal Filter. Then, 6 mL of PBS was added from the top, centrifuged at 2500g for 30 minutes at 4-8°C, and the waste liquid was removed from the bottom of the Amicon^{®} Ultra Centrifugal Filter. Then, the absorbance at 280 nm (OD280) was measured to calculate the concentration, then the antibody concentration was adjusted to 5mg/mL with PBS, and the solution was mixed with 62.5% glycerol in a volume of 1 (antibody):4 (glycerol) and stored at -20°C to obtain a final antibody concentration of 1mg/mL for subsequent use.

### Step 5:

1 mL of Frozen Human NK cells (TCI GENE; Lot.no. 20211014) and 10 mL of PBS were transferred to a 15 mL centrifuge tube, centrifuged and washed at 400g for 5 minutes, and transferred to a 10 cm cell culture dish with 10 mL of NK culture medium (RPMI culture medium, 5% EG, 500 IU/mL rhIL-2) for incubation. After incubating at 37°C, 5% CO₂ for one day, the NK cells were washed and the buffer was replaced using the following methods (A) or (B):
(A) The NK cells were transferred to a 15 mL centrifuge tube, centrifuged at 400g at 4-8°C for 5 minutes, the supernatant was removed, then the NK cells were washed twice with 10 mL PBS, and finally NK cells were resuspended in PBS and the NK cell concentration was adjusted to 2×10⁶ cells/mL using PBS.
(B) The NK cells were transferred to a 50 mL centrifuge tube, centrifuged at 400g at 4-8°C for 5 minutes, the supernatant was removed, then the NK cells were washed once with 50 mL PBS, and finally NK cells were resuspended in PBS and the NK cell concentration was adjusted to 2×10⁶ cells/mL using PBS. Finally, 1 mL of the resulting solution was distributed into each of the 3 tubes and labeled as the control group, comparison group, and experimental group, respectively.

### Step 6:

### [Preparation of Control Group, Comparison Group, and Experimental Group]

The control group was a mixture formed by adding 1 mL of PBS and 1 mL of NK cell solution; the comparison group was prepared by taking 900 µL of PBS and adding 100 µL of 1 mg/mL linker conjugated Atezolizumab solution; the experimental group was prepared by taking 900 µL of PBS and adding 100 µL of 1 mg/mL linker conjugated Atezolizumab solution. Then, the resulting solution was mixed with 1 mL of NK cell solution from Step 5 at a of volume 1:1 to make the final Atezolizumab concentration of 50 µg/mL and the NK cell concentration of 1×10⁶ cells/mL. Then, the solution was uniformly irradiated with UV at a wavelength of 365 nm with 400 mJ/cm², and the mixtures of the control group, comparison group, and experimental group were allowed to stand at 2-8°C for 10 minutes.

### Step 7:

The mixtures from Step 6 were divided into the following two groups for subsequent experiments:
(A) For small-scale cell test: The mixtures from Step 2 were transferred to a 15 mL centrifuge tube and an equal volume of PBS/1%HSA solution was added. After the resulting solutions were centrifuged at 400g for 5 minutes at 4-8°C, the supernatant was removed, then the NK cells were washed with 10 mL PBS/1%HSA twice, and the NK cells were resuspended in the solution required for the experiment and the NK cell concentration was adjusted to 2×10⁶ cells/mL.
(B) For large-scale animal test: The mixtures from Step 2 were transferred to a 50 mL centrifuge tube and an equal volume of PBS/1%HSA solution was added. After the resulting solutions were centrifuged at 400g for 5 minutes at 4-8°C, the supernatant was removed, then the NK cells were washed with 50 mL PBS/1%HSA once, and the NK cells were resuspended in the solution required for the experiment and the NK cell concentration was adjusted to 2×10⁶ cells/mL.

### Step 8:

After 200 µL of the solution from each group in Step 7 was taken into 1.5 mL test tube (Eppendorf) tube and Goat F(ab')2 Anti-Human IgG-(Fab')2 (PE) (abcam, cat.no. ab98606) was diluted in PBS at a volume ratio of 1:100, 200 µL of this dilution was added to solutions of each group from Step 6, and reacted at 4°C for 30 minutes. Then, 800 µL of PBS was added, the mixture was centrifuged at 400g for 5 minutes at 4-8°C, the supernatant was removed, then the cells were washed twice with 1 mL PBS, and the supernatant was removed. Finally, the cells were resuspended in 200 µL PBS for each group, and analyzed by flow cytometry (FACS).

### Experimental Results:

Goat F(ab')2 Anti-Human IgG-(Fab')2 (PE) was used to carry out antibody staining on the NK cell membrane, followed by flow cytometry (FACS) analysis of the PE signal to obtain the strength of antibody conjugating to NK cells.

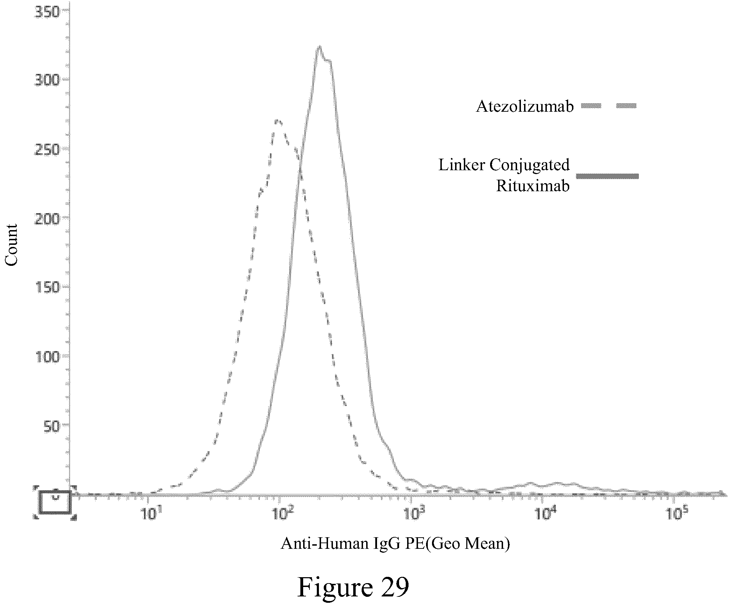

Figure 29 shows the conjugating results of linker conjugated Atezolizumab to NK cells. From Figure 29, it can be observed that the linker conjugated Atezolizumab in the experimental group was successfully conjugated to the surface of NK cells.

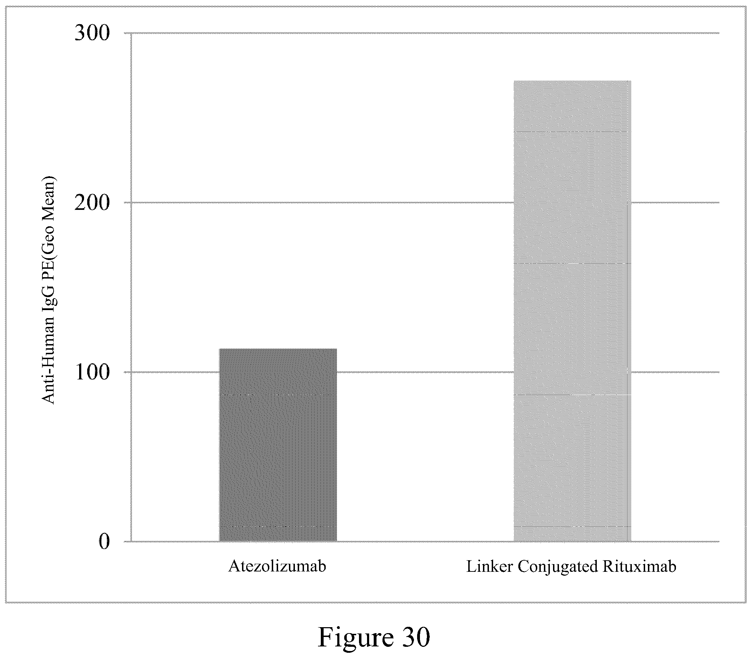

Figure 30 shows the conjugating ability of linker conjugated Atezolizumab to NK cells. From Figure 30, it can be observed that, compared to Atezolizumab in the comparison group, the linker (formula α) conjugated Atezolizumab in the experimental group had a better conjugating ability to NK cells. Thus, the experiment demonstrated that the strategy of using a linker to conjugate antibodies to cells can be extended to other antibodies beyond Trastuzumab.

### Example 17: [The Results of Cytotoxicity Test of Rituximab Conjugated PBMC Cells]

The following describes the steps of the cytotoxicity test of Rituximab conjugated PBMC cells:

### Step 1:

After Human Burkitt's lymphoma cells (Daudi cells) were incubated in a T75 culture flask with 15 mL of cell culture medium (90% RPMI 1640 culture medium and 2 mM L-glutamine, adjusted to include 1.5 g/L sodium bicarbonate, 4.5 g/L glucose, 10 mM 4-(2-hydroxyethyl)-1-piperazineethanesulfonic acid (HEPES), and 1.0 mM sodium pyruvate + 10% FBS) at 37°C in a 5% CO₂ incubator for 3 days, the Daudi cells were transferred to a 50 mL centrifuge tube, centrifuged at 400g for 5 minutes at 4-8°C, then the supernatant was removed, and then the cells were washed twice with PBS. Finally, the Daudi cells were resuspended in PBS and the cell concentration was adjusted to 5×10⁶-1 ×10⁷ cells/mL.

### Step 2:

2 mL of Daudi cells were mixed with 2 mL of 1 µM CellTracker^{™} Deep Red solution at a volume ratio of 1:1, making a total volume of 4 mL, and the mixture was incubated at room temperature, protected from light, for 10 minutes, and then centrifuged and washed twice with PBS. Finally, the Daudi cells were resuspended in PBS and the cell concentration was adjusted to 1×10⁵ cells/mL.

### Step 3:

The PBMC cell suspension was prepared at 2×10⁶ cells/mL according to Step 1 of Example 12.

### Step 4:

### [Preparation of Control Group, Comparison Group, and Experimental Group]

The control group was a mixture formed by adding 1 mL of PBS and 1 mL of PBMC cell solution; the comparison group was prepared by taking 900 µL of PBS and adding 100 µL of 1 mg/mL Rituximab solution; the experimental groups was prepared by taking 900 µL of PBS and adding 100 µL of 1 mg/mL of linker formula α conjugated Rituximab solution. The solutions were uniformly irradiated with UV at a wavelength of 365 nm for 10 seconds, followed by a standing period of 10 seconds, repeated twice in total, then 1 mL of the PBMC solution from Step 3 was added, resulting in a mixture with a final Rituximab antibody concentration of 50 µg/mL and a mixture with a final concentration of linker formula α conjugated Rituximab of 50 µg/mL. Then, the mixtures of the control group, comparison group, and experimental group were allowed to stand at 4°C for 10 minutes.

### Step 5:

After the mixtures from Step 4 were centrifuged at 400g for 5 minutes at 4-8°C, the supernatant was removed, then the cells were washed twice with 10 mL PBS, and finally the cells were resuspended in Daudi cell culture medium and the cell concentration was adjusted to 1×10⁶ cells/mL for subsequent experiments.

### Step 6:

The PBMC cells from the experimental group, control group, and comparison group were mixed with Daudi cells stained with CellTracker^{™} Deep Red at ratios of 1:1, 5:1, and 10:1, respectively, into a 96-well flat-bottom plate to a final volume of 200 µL, and reacted at 37°C for 4 hours.

### Step 7:

200 µL of the cytotoxic effect cells after 4 hours of reaction were mixed with 100 µL of Annexin V/PI solution, reacted at room temperature, protected from light, for 5 minutes, and analyzed the Deep Red⁺/Annexin V⁺/PI⁺ cell population by flow cytometry (FACS).

### Experimental Results:

First, the Deep Red⁺ Daudi cell population was selected, then the cell proportion of Annexin V⁺/PI⁺ Daudi cells was analyzed, then the value of spontaneous cell death (Annexin V⁺/PI⁺) in Daudi cells was subtracted to compare the NK cytotoxicity (specific lysis) effect.

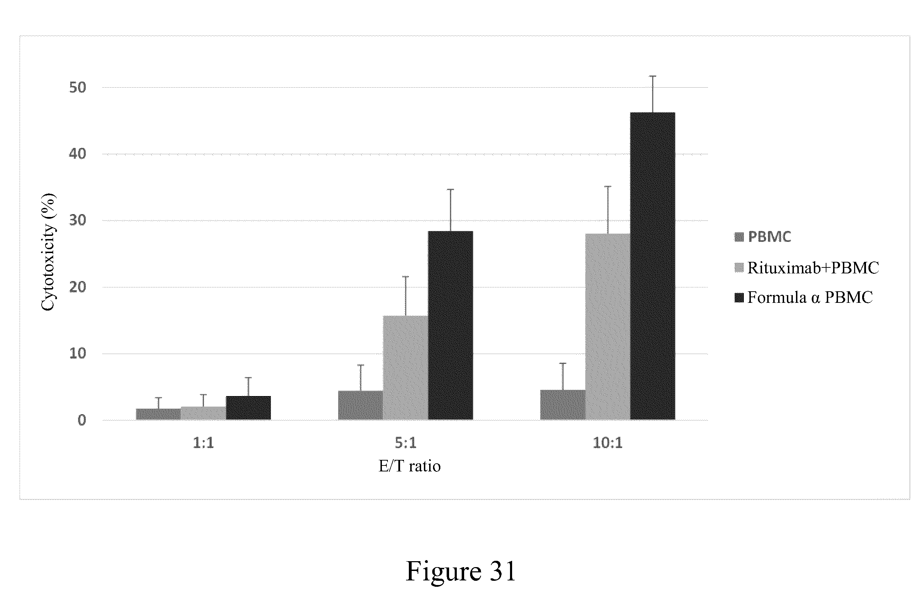

Figure 31 shows the cytotoxicity test results of Rituximab conjugated-PBMC cells. From Figure 31, it can be observed that, compared to PBMC in the control group and Rituximab+PBMC in the comparison group, the PBMC cells of linker conjugated antibody in the experimental group (formula α PBMC) exhibited greater cytotoxic effects with E/T ratios (effector to target ratio) of 5:1 and 10:1, with cytotoxic effects of 12.67% and 26.06%, respectively, and the cytotoxic effect increased along with the PBMC cell ratio.

### Example 18: [Efficacy of Linker Conjugated Trastuzumab-NK Cells in a Breast Cancer Xenograft Model]

The following describes the steps of the efficacy of linker conjugated Trastuzumab-NK cells in a breast cancer xenograft model.

### Step 1:

The subcutaneous BT-474 (passage p3-p6 after thawing) xenograft tumor model was established in NOD-SCID mice to evaluate the efficacy of linker conjugated Trastuzumab. The three groups consisted of the vehicle comparison (n=8), the comparison (n=7), and the experimental group (n=7), which contained normal saline with 1% human serum albumin (HSA), NK cells, and linker conjugated Trastuzumab-NK, respectively. The BT-474 xenograft tumor model was established in NOD-SCID mice (female, nulliparous, and non-pregnant), and estradiol particles were implanted in the back of each mouse. Seven days before drug administration, 2.5 × 10⁶ BT-474 cells/mouse were subcutaneously seeded (the BT-474 cells were suspended in a 1:1 mixture of 0.1 mL 1x DPBS and 0.1 mL Matrigel). Before the start of the study, the animals were 7 weeks old, and their weight variation did not exceed ±20% of the average weight for each sex.

### Step 2:

The linker formula α conjugated Trastuzumab was prepared according to Example 4.

### Step 3:

6 mL of Frozen Human NK cells (TCI GENE; Lot.no. 20211014) and 18 mL of PBS (containing 1% HSA) were transferred to a 50 mL centrifuge tube, after being centrifuged at 400g for 5 minutes, the supernatant was removed. Then, after the NK cells were centrifuged and washed with PBS (containing 1% HSA), the supernatant was removed, replaced with 40 mL NK culture medium (RPMI culture medium, 5% EG, 500 IU/mL rhIL-2) and the NK cell concentration was adjusted to 5×10⁵ cells/mL, and then 20 mL of the cell suspension was transferred to a T75 culture flask for incubation. After two days of incubation at 37°C, 5% CO₂, the NK cells were transferred to a 50 mL centrifuge tube, and the T75 culture flask was washed with 5 mL PBS, and the solution was transferred to the 50mL centrifuge tube, then after being centrifuged at 400g for 9 minutes at 4-8°C, the supernatant was removed, and the NK cells were washed twice with PBS. Finally, the NK cells were resuspended in PBS and divided into two groups, labeled as the comparison group and the experimental group, respectively, and the NK cell concentration in the experimental group was adjusted to 2×10⁶ cells/mL.

### Step 4:

### [Preparation of Comparison Group and Experimental Groups]

The control group NK cell suspension was added to normal saline containing 1% HSA, after being centrifuged at 400g for 5 minutes at 4-8°C, the supernatant was removed, and the NK cells were resuspended in normal saline containing 1% HSA, and the concentration of comparison group NK cells was adjusted to 1×10⁷ cells/mL; 27.9 mL of PBS was taken and mixed with 2.1 mL of 1 mg/mL linker conjugated Trastuzumab solution. The solution was uniformly irradiated with UV at a wavelength of 365 nm for 10 seconds, followed by a standing period of 10 seconds, repeated twice in total, then 1 mL of the NK cell solution in the experimental group from Step 2 was added, resulting in a mixture with final concentration of linker formula α conjugated Trastuzumab of 35 µg/mL, and the mixtures of the experimental group were allowed to stand at 4°C for 15 minutes.

### Step 5:

After the mixtures from Step 4 were centrifuged at 400g for 5 minutes at 4-8°C, the supernatant was removed, and the cells were washed twice with PBS, and finally the cells were resuspended in normal saline containing 1% HSA and the NK cell concentration was adjusted to 1×10⁷ cells/mL. It was confirmed that the conjugation rate of linker formula α conjugated Trastuzumab to NK cells was ≥70% and the NK purity (CD3⁺CD56⁺) was ≥50%.

### Step 6:

The drugs were administered intraperitoneally at 0.5 mL/mouse on the 1st day, 4th day, 7th day, 11th day, and 14th day, repeated five times in total. Mice identified as having non solid tumors upon dissection were excluded from the results of this study. The data of the analysis data were shown as mean±SEM; single-factor variance analysis (One way ANOVA) was conducted using the t-test (Cochran-Cox test); *p < 0.05. The formula for tumor growth inhibition (TGI) was: (average tumor size of vehicle comparison group - individual tumor size) / (average tumor size of vehicle comparison group) × 100%.

### Experimental Results:

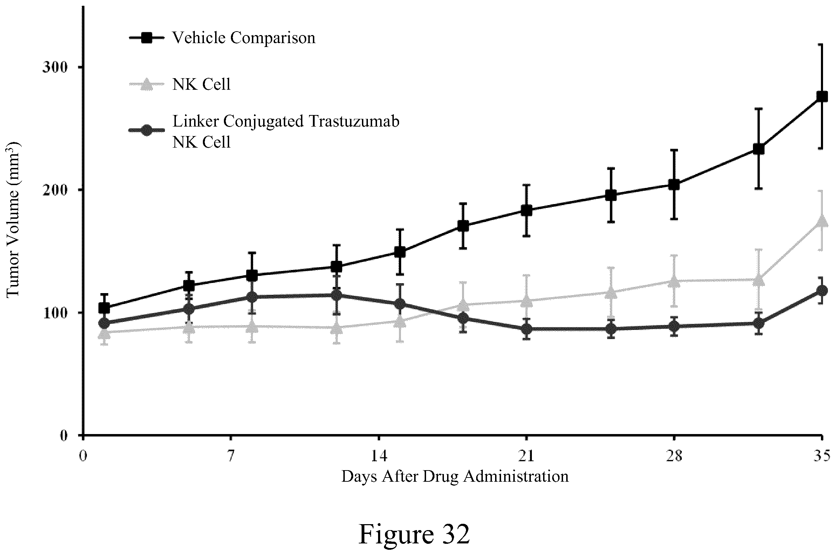

Figure 32 shows the tumor volume measurement results. From Figure 32, it can be observed that from the 18th day to the 35th day after drug administration, the experimental group (linker conjugated Trastuzumab NK cells, n=6) inhibited tumor growth, resulting in the tumor volume starting to shrink. On the 35th day, the tumor volumes in the vehicle comparison group, NK cell comparison group, and experimental group were 276.1 ± 51.1 mm³, 175.1 ± 40.9 mm³, and 118 ± 15.2 mm³, respectively, indicating that the experimental group had a significantly smaller tumor volume.

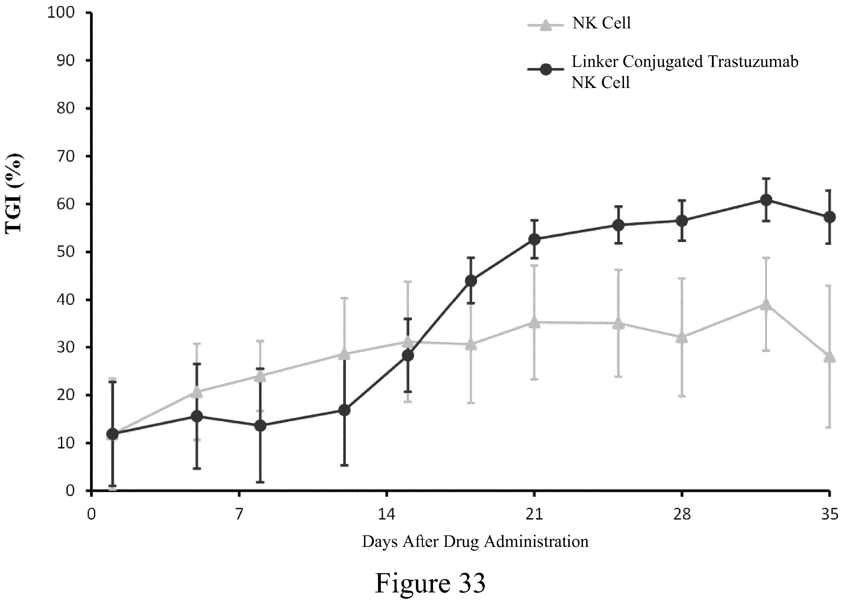

Figure 33 shows the tumor growth inhibition (TGI) analysis results. From Figure 33, it can be observed that, compared to the vehicle comparison group (normal saline containing 1% HSA, n=6), the experimental group (linker conjugated Trastuzumab NK cells, n=6) showed a statistically significant tumor growth inhibition rate (57.3±5.5%, p < 0.05), and the comparison group (NK cells, n=6) showed no statistically significant tumor growth inhibition (36.6±14.8%). From this, it can be observed that the linker conjugated Trastuzumab NK cells significantly enhanced anti-tumor efficacy, and demonstrated their potential to be translated into human clinical trials and linker-conjugated antibodies to be applied in the field of cell therapy.

### Example 19 [Analysis of the Cytotoxicity of Linker Conjugated Trastuzumab-NK Cells Against N87 Gastric Cancer Cells]

The following describes the steps for comparing the cytotoxicity of linker conjugated Trastuzumab-NK cells against N87 gastric cancer cells.

### Step 1:

N87 gastric cancer cells were incubated in a 10 cm culture dish with 10 mL of cell culture medium (IMDM/10% FBS/30ng/mL EGF) at 37°C, 5% CO₂ for three days. The cell medium was removed, and then the cells were washed with 10 mL PBS. 1 mL of CTS^{™} TrypLE^{™} Select enzyme was added and reacted at 37°C, 5% CO₂ for 5 minutes. Then, 10 mL of cell culture medium was added to collect the cells, which were transferred to a 15 mL centrifuge tube, and then after being centrifuged at 400g for 5 minutes at 4-8°C, the supernatant was removed, and the cells were washed and centrifuged twice with PBS, and finally the N87 gastric cancer cells were resuspended in PBS and the cell concentration was adjusted to 5×10⁶-1×10⁷ cells/mL.

### Step 2:

20 µL of DMSO solvent was added to a Deep Red reagent tube, 5 µL of Deep Red solution was taken and added to 5 mL of PBS to prepare a 1 µM CellTracker^{™} Deep Red dye (Invitrogen^{™}, C34565) solution, and stored and protected from light. 2 mL of N87 gastric cancer cells were mixed with 2mL of 1 µM Deep Red solution at a 1:1 volume ratio, for a total of 4mL, and then reacted at room temperature, protected from light, for 10 minutes.

### Step 3:

The Deep Red-stained H2170 cells were centrifuged and washed twice with 10 mL of cell culture medium (IMDM/10%FBS/30ng/ml. EGF) at 400g for 5 minutes at 4-8°C. The cell concentration of Deep Red-stained N87 gastric cancer cells was adjusted to 1×10⁵ cells/mL using cell culture medium.

### Step 4:

The NK cells in the experimental group, control group, and comparison group from Example 6 were taken and mixed with CellTracker^{™} Deep Red-stained H2170 cells at ratios of 1:1, 5:1, and 10:1, respectively, to a final volume of 200 µL, then placed to a 96-well flat-bottom plate, and then reacted at 37°C for 4 hours

### Step 5:

100 µL of Annexin V/PI solution was prepared (98.5 µL of 1X binding buffer was added with 1 µL of Annexin V and 0.5 µL of PI). 200 µL of the cytotoxic effect cells after the 4 hours of reaction were taken and mixed with 100 µL of Annexin V/PI solution, reacted at room temperature, protected from light, for 5 minutes, and analyzed for the Deep Red⁺/Annexin V⁺/PI⁺ cell population by flow cytometry (FACS).

### Experimental Results:

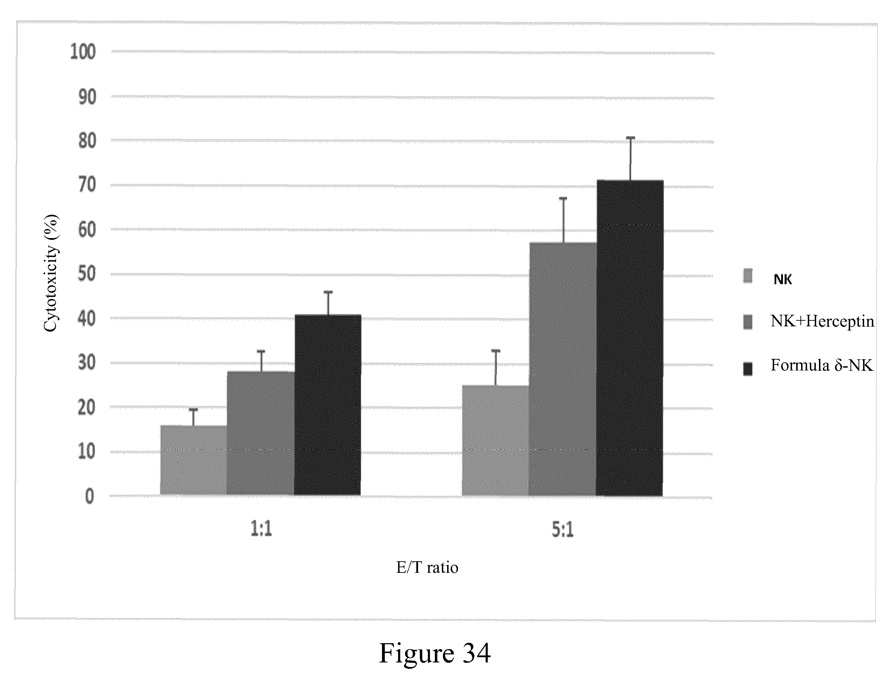

Figure 34 shows the analysis results of the cytotoxicity of linker conjugated Trastuzumab-NK cells against N87 gastric cancer cells. From Figure 34, it can be observed that, compared to the NK cells in the control group and the NK+Herceptin in the comparison group, the NK cells conjugated with linker conjugated Trastuzumab (formula δ-NK) exhibited a greater cytotoxic effect against N87 gastric cancer cells, with cytotoxicity rates of 41%+5.2% and 71.6%+9.4% at E/T ratios of 1:1 and 5:1, respectively, and the cytotoxic effect increased along with the NK cell ratio.

### Example 20 [Analysis of the Cytotoxicity of Linker Conjugated Trastuzumab-NK Cells Against H2170 Lung Cancer Cells]

The following describes the steps for comparing the cytotoxicity of linker conjugated Trastuzumab-NK cells against H2170 lung cancer cells.

### Step 1:

H2170 lung cancer cells were incubated in a 10 cm cell culture dish with 10 mL of cell culture medium (IMDM/10% FBS/30ng/mLEGF) at 37°C, 5% CO₂ for three days. The cell medium was removed and the cells were washed with 10 mL PBS, and then 1 mL of CTS^{™} TrypLE^{™} Select enzyme was added and reacted at 37°C, 5% CO₂ for 5 minutes. Then, 10 mL of cell culture medium was added to collect the cells, which were transferred to a 15mL centrifuge tube, and after being centrifuged at 400g for 5 minutes at 4-8°C, the supernatant was removed, and then the H2170 lung cancer cells were washed and centrifuged twice with PBS, and finally H2170 lung cancer cells were resuspended in PBS and the cell concentration were adjusted to 5×10⁶-1×10⁷ cells/mL.

### Step 2:

20 µL of DMSO solvent was added to a Deep Red reagent tube, 5 µL of Deep Red solution was taken and added to 5 mL of PBS to prepare a 1 µM CellTracker^{™} Deep Red dye (Invitrogen^{™}, C34565) solution, which was stored and protected from light. 2 mL of H2170 lung cancer cells were mixed with 2 mL 1 µM Deep Red solution at a volume ratio of 1:1, for a total of 4 mL, and then incubated at room temperature, protected from light, for 10 minutes.

### Step 3:

The Deep Red-stained H2170 lung cancer cells were centrifuged and washed twice with 10 mL of cell culture medium (IMDM/10% FBS/30ng/mL EGF) at 400g for 5 minutes at 4-8°C. The cell concentration of Deep Red-stained H2170 lung cancer cells was adjusted to 1×10⁵ cells/mL using cell culture medium.

### Step 4:

After NK cells in the experimental group, control group, and comparison group from Example 6 were taken and mixed with CellTracker^{™} Deep Red-stained H2170 lung cancer cells at ratios of 1:1, 5:1, and 10:1, respectively, to a final volume of 200 µL, placed to a 96-well flat-bottom plate, and then reacted at 37°C for 4 hours.

### Step 5:

100µL of Annexin V/PI solution was prepared (98.5 µL of 1X binding buffer was added with 1 µL Annexin V and 0.5 µL PI). 200 µL of the cytotoxic effect cells after the 4 hours of reaction were taken and mixed with 100 µL of Annexin V/PI solution, after reacted at room temperature, protected from light, for 5 minutes, analyzed the Deep Red⁺/Annexin V⁺/PI⁺ cell population by flow cytometry (FACS).

### Experimental Results:

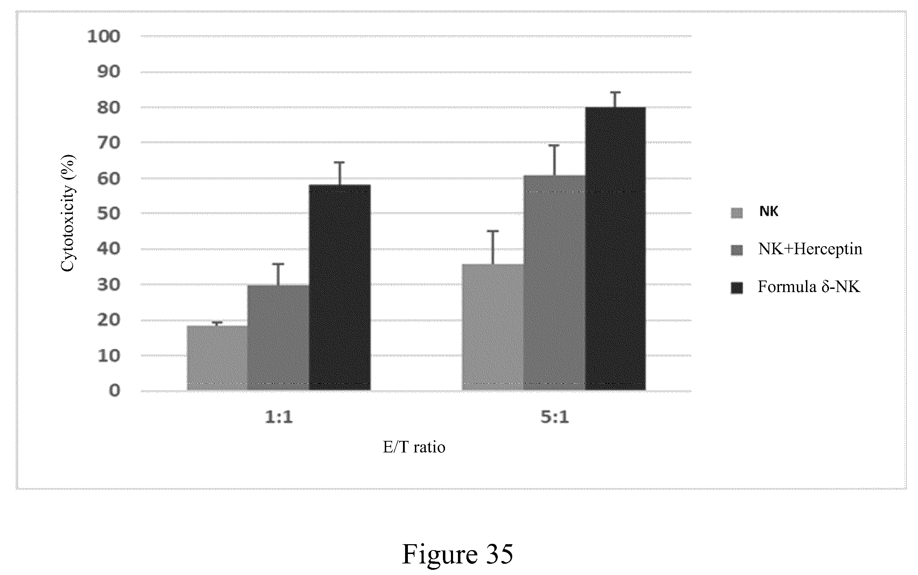

Figure 35 shows the analysis results of the cytotoxicity of linker conjugated Trastuzumab-NK cells against H2170 lung cancer cells. From Figure 35, it can be observed that, compared to the NK cells in the control group and the NK+Herceptin in the comparison group, the NK cells conjugated with linker conjugated Trastuzumab (formula δ-NK) exhibited a greater cytotoxic effect against H2170 lung cancer cells, with cytotoxicity rates of 58.3% ± 6.2% and 79.9% ± 4.3% at E/T ratios of 1:1 and 5:1, respectively, and the cytotoxic effect increased along with the NK cell ratio.

### Example 21: [Analysis of UV Irradiation Energy on the Conjugating Degree of Different Hydrocarbon Chain Linker Conjugated Trastuzumab to NK Cells]

The following describes the steps for comparing the conjugating degree of different hydrocarbon chain linker conjugated Trastuzumab to NK cells under various UV 365 nm irradiation energies:

### Step 1:

Four tubes containing 5mL(2mg/mL) Herceptin antibody (Roche, Trastuzumab) were taken, and 67.55 µL of 10 mM linker formula α solution, 67.55 µL of 10 mM linker formula δ solution, and 101.3 µL of 10 mM NHS-Diazirine solution were added to each, respectively. Then the solutions were mixed and reacted at room temperature for 30 minutes or at 2-8°C for 3 hours to generate linker formula α-T solution, linker formula δ-T solution, and Diazirine-T solution.

### Step 2:

The different hydrocarbon chain linker conjugated Trastuzumab solutions were transferred into an Amicon^{®} Ultra Centrifugal Filter, then 11 mL of PBS was added, and centrifuged at 2500g for 30 minutes at 4-8°C. The waste liquid was removed from the bottom of the Amicon^{®} Ultra Centrifugal Filter, and 6 mL of PBS was added from the top, centrifuged at 2500g for 30 minutes at 4-8°C. The waste liquid was removed from the bottom of the Amicon^{®} Ultra Centrifugal Filter, then absorbance OD280 was measured to calculate the concentration, the antibody concentration was adjusted to 5 mg/mL with PBS, and the resulting solution was mixed with 62.5% glycerol at a volume ratio of 1 (antibody):4 (glycerol) and stored at -20°C.

### Step 3:

### [Preparation of Control Group, Comparison Group, and Experimental Groups]

1mL of Frozen Human NK cells (TCI GENE; Lot.no. 20210816) and 10 mL PBS were transferred to a 15 mL centrifuge tube, centrifuged at 400g for 5 minutes and the NK cell concentration was adjusted to 5×10⁵ cells/mL using NK culture medium (RPMI medium, 5% EliteGro, 500 IU/mL rhIL-2), and transferred to a T75 culture flask for incubation. After incubation at 37°C, 5% CO₂ for one day, the NK cells were transferred to a 50 mL centrifuge tube, after being centrifuged at 400g at 4-8°C for 5 minutes, the supernatant was removed, and then the NK cells were washed twice with 50 mL PBS. Finally, the NK cells were resuspended in PBS and the NK cell concentration was adjusted to 2×10⁶ cells/mL. 0.3 mL per tube was distributed, and mixed with Trastuzumab solution (60 µg/mL), Diazirine-T solution (60 µg/mL), formula α-T solution (60 µg/mL), and formula δ-T solution (60 µg/mL), respectively. The mixtures were labeled as the control group (no UV irradiation), comparison group (Trastuzumab and Diazirine-T), and experimental group (formula α-T and formula δ-T). The mixtures were irradiated at 365 nm UV with 100, 200, 300, 400, and 500 mJ/cm², then placed at 2-8°C to stand for 10 minutes, and blocking solution (1% human serum albumin/RPMI medium) was added.

### Step 4:

After the mixtures from Step 3 were centrifuged at 400g for 5 minutes at 4-8°C, the supernatant was removed, the cells were washed twice with 10 mL PBS, and finally the cells were resuspended in PBS and the cell concentration was adjusted to 5×10⁵ cells/mL for subsequent experiments.

### Step 5:

After 200 µL of solutions of each group from Step 4 was taken into 1.5 mL test tube (Eppendorf). Goat F(ab')2 Anti-Human IgG-(Fab')2 (PE) (abcam, cat.no. ab98606) was diluted in PBS at a volume ratio of 1:200, and 200 µL of this dilution was added to solutions of each group, and reacted at 4°C for 15-30 minutes. 1000µL PBS was added, after being centrifuged at 400g for 5 minutes at 4-8°C, the supernatant was removed, then the cells were washed and centrifuged twice with 1 mL PBS, the supernatant was removed, the cells were resuspended in 200µL PBS for each group, and analyzed by flow cytometry (FACS).

Finally, Goat F(ab')2 Anti-Human IgG-(Fab')2 (PE) and CD16 monoclonal antibody were used to carry out antibody staining on the NK cell membrane, followed by flow cytometry analysis of the PE signal to obtain the strength of Trastuzumab conjugating to NK cells.

### Experimental Results:

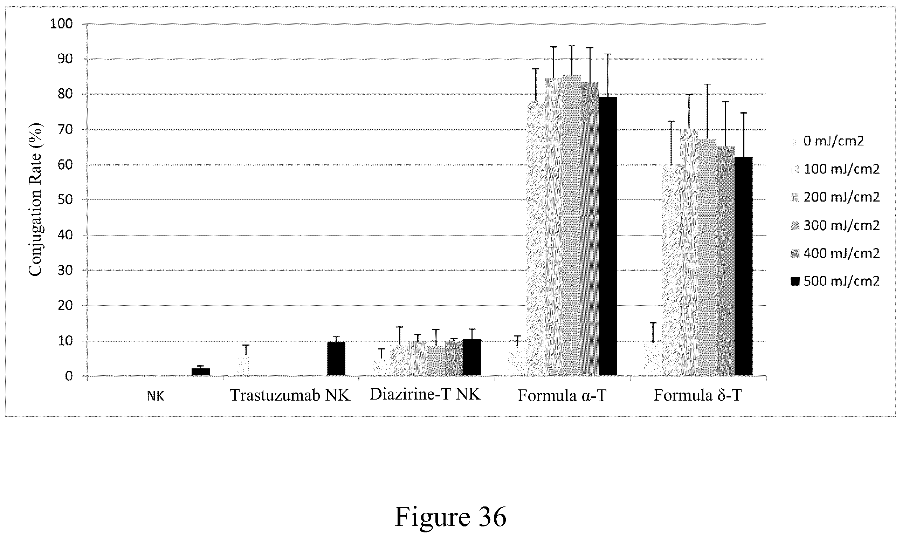

Figure 36 shows the analysis results of the conjugating degree of linker conjugated Trastuzumab to NK cells under different UV irradiation energies. From Figure 36, it can be observed that after 365 nm UV irradiation, compared to the Trastuzumab NK and the Diazirine-T NK in the commercially available photosensitive molecule in comparison group, the formula α-T NK and formula δ-T NK in the experimental group were successfully conjugated to the surface of NK cells. The formula α-T and formula δ-T exhibited higher conjugating efficiency when irradiated with 365 nm UV at energy intensities of 200-400 mJ/cm².

### Example 22 [Analysis of the Conjugating Degree of Different Hydrocarbon Chain Linker Conjugated Trastuzumab to NK Cells at Various Reaction Times after UV Irradiation]

The following describes the steps for comparing the conjugating degree of different hydrocarbon chain linker conjugated Trastuzumab to NK cells under 365 nm UV irradiation at 400 mJ/cm² and various reaction times at low temperature:

### Step 1:

Four tubes containing 5mL(2mg/mL) Herceptin antibody (Roche, Trastuzumab) were taken, and 67.55 µL of 10 mM linker formula α solution, 67.55 µL of 10 mM linker formula δ solution, and 101.3 µL of 10 mM NHS-Diazirine solution were added to each, respectively. Then the solutions were mixed and reacted at room temperature for 30 minutes or at 2-8°C for 3 hours to generate linker formula α-T solution, linker formula δ-T solution, and Diazirine-T solution.

### Step 2:

The different hydrocarbon chain linker conjugated Trastuzumab solutions were transferred into an Amicon^{®} Ultra Centrifugal Filter, then 11 mL of PBS was added, and centrifuged at 2500g for 30 minutes at 4-8°C. The waste liquid was removed from the bottom of the Amicon^{®} Ultra Centrifugal Filter, and 6 mL of PBS was added from the top, centrifuged at 2500g for 30 minutes at 4-8°C. The waste liquid was removed from the bottom of the Amicon^{®} Ultra Centrifugal Filter, then absorbance OD280 was measured to calculate the concentration, the antibody concentration was adjusted to 5 mg/mL with PBS, and the resulting solution was mixed with 62.5% glycerol at a volume ratio of 1 (antibody) : 4 (glycerol) and stored at -20°C.

### Step 3:

### [Preparation of Control Group, Comparison Group, and Experimental Groups]

1mL of Frozen Human NK cells (TCI GENE; Lot.no. 20210816) and 10mL PBS were transferred to a 15 mL centrifuge tube, centrifuged at 400g for 5 minutes and the NK cell concentration was adjusted to 5×10⁵ cells/mL using NK culture medium (RPMI medium, 5% EliteGro, 500 IU/mL rhIL-2), and transferred to a T75 culture flask for incubation. After incubation at 37°C, 5% CO₂ for one day, the NK cells were transferred to a 50 mL centrifuge tube, after being centrifuged at 400g at 4-8°C for 5 minutes, the supernatant was removed, and then the NK cells were washed twice with 50 mL PBS. Finally, the NK cells were resuspended in PBS and the NK cell concentration was adjusted to 2×10⁶ cells/mL. 0.3 mL per tube was distributed, and mixed with Trastuzumab solution (60 µg/mL), Diazirine-T solution (60 µg/mL), formula α-T solution (60 µg/mL), and formula δ-T solution (60 µg/mL), respectively. The mixtures were labeled as the control group (no low-temperature reaction), comparison group (Trastuzumab and Diazirine-T), and experimental group (formula α-T and formula δ-T). The mixtures were irradiated at 365 nm UV with 400 mJ/cm², then placed at 2-8°C to stand for 1, 5, 10, and 15 minutes, respectively, and blocking solution (1% human serum albumin/RPMI medium) was added.

### Step 4:

After the mixtures from Step 3 were centrifuged at 400g for 5 minutes at 4-8°C, the supernatant was removed, the cells were washed twice with 10 mL PBS, and finally the cells were resuspended in PBS and the cell concentration was adjusted to 5×10⁵ cells/mL for subsequent experiments.

### Step 5:

After 200 µL of solutions of each group from Step 4 was taken into 1.5 mL test tube (Eppendorf). Goat F(ab')2 Anti-Human IgG-(Fab')2 (PE) (abcam, cat.no. ab98606) was diluted in PBS at a volume ratio of 1:200, and 200 µL of this dilution was added to solutions of each group, and reacted at 4°C for 15-30 minutes. 1000µL PBS was added, after being centrifuged at 400g for 5 minutes at 4-8°C, the supernatant was removed, then the cells were washed and centrifuged twice with 1 mL PBS, the supernatant was removed, the cells were resuspended in 200µL PBS for each group, and analyzed by flow cytometry (FACS).

Finally, Goat F(ab')2 Anti-Human IgG-(Fab')2 (PE) and CD16 monoclonal antibody were used to carry out antibody staining on the NK cell membrane, followed by flow cytometry analysis of the PE signal to obtain the strength of Trastuzumab conjugating to NK cells.

### Experimental Results:

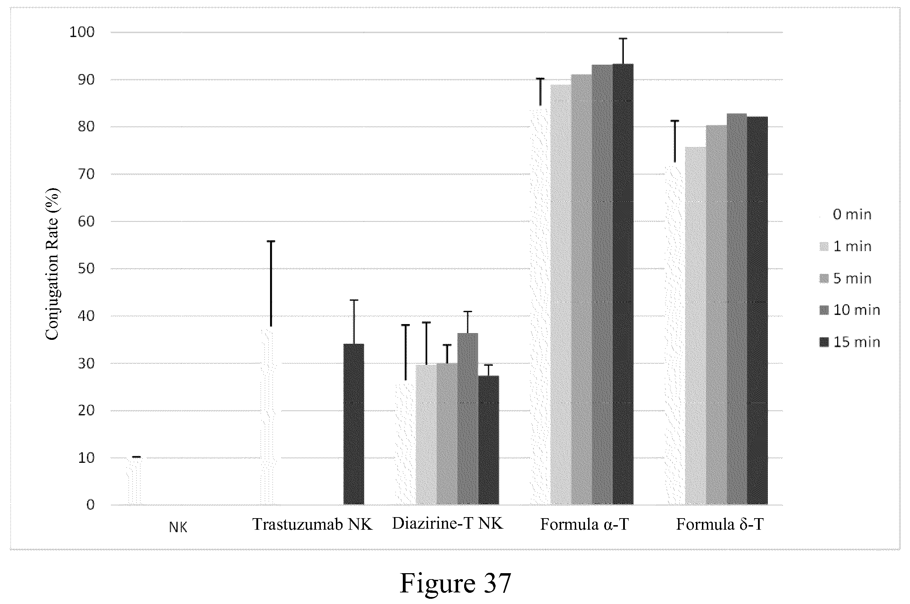

Figure 37 is a diagram that shows the analysis results of the conjugating degree of different linker conjugated Trastuzumab against to NK cells at different reaction times after UV irradiation. From Figure 37, it can be observed that after 365 nm UV irradiation, compared to the Trastuzumab NK and the Diazirine-T NK in the commercially available photosensitive molecule in comparison group, the formula α-T NK and formula δ-T NK in the experimental group were successfully conjugated to the surface of NK cells, in which formula α-T and formula δ-T exhibited higher conjugating degree with NK cells when reacted at low temperature for 5 to 15 minutes.

### Example 23 [Analysis of the Conjugating Degree and the Cytotoxicity Results of different hydrocarbon chain linker conjugated Trastuzumab at various antibody concentrations to NK cells]

The following describes the steps for comparing the NK cell conjugating degree and the cytotoxicity results of different hydrocarbon chain linker conjugated Trastuzumab under various antibody concentrations after UV irradiation:

### Step 1:

Four tubes containing 5mL(2mg/mL) Herceptin antibody (Roche, Trastuzumab) were taken, and 67.55 µL of 10 mM linker formula α solution, 67.55 µL of 10 mM linker formula δ solution, and 101.3 µL of 10 mM NHS-Diazirine solution were added to each, respectively. Then the solutions were mixed and reacted at room temperature for 30 minutes or at 2-8°C for 3 hours to generate linker formula α-T solution, linker formula δ-T solution, and Diazirine-T solution.

### Step 2:

The different hydrocarbon chain linker conjugated Trastuzumab solutions were transferred into an Amicon^{®} Ultra Centrifugal Filter, then 11 mL of PBS was added, and centrifuged at 2500g for 30 minutes at 4-8°C. The waste liquid was removed from the bottom of the Amicon^{®} Ultra Centrifugal Filter, and 6 mL of PBS was added from the top, centrifuged at 2500g for 30 minutes at 4-8°C. The waste liquid was removed from the bottom of the Amicon^{®} Ultra Centrifugal Filter, then absorbance OD280 was measured to calculate the concentration, the antibody concentration was adjusted to 5 mg/mL with PBS, and the resulting solution was mixed with 62.5% glycerol at a volume ratio of 1 (antibody) : 4 (glycerol) and stored at -20°C.

### Step 3:

### [Preparation of Control Group, Comparison Group, and Experimental Groups]

1mL of Frozen Human NK cells (TCI GENE; Lot.no. 20210816) and 10mL PBS were transferred to a 15 mL centrifuge tube, centrifuged at 400g for 5 minutes and the NK cell concentration was adjusted to 5×10⁵ cells/mL using NK culture medium (RPMI medium, 5% EliteGro, 500 IU/mL rhIL-2), and transferred to a T75 culture flask for incubation. After incubation at 37°C, 5% CO₂ for one day, the NK cells were transferred to a 50 mL centrifuge tube, after being centrifuged at 400g at 4-8°C for 5 minutes, the supernatant was removed, and then the NK cells were washed twice with 50 mL PBS. Finally, the NK cells were resuspended in PBS and the NK cell concentration was adjusted to 2×10⁶ cells/mL. 0.3 mL per tube was distributed, and mixed with Trastuzumab solution (60 µg/mL), Diazirine-T solution (60 µg/mL), formula α-T solution (20, 40, 60 µg/mL), and formula δ-T solution (20, 40, 60 µg/mL), respectively. The mixtures were labeled as the control group (NK only), comparison group (Trastuzumab and Diazirine-T), and experimental group (formula α-T and formula δ-T). The mixtures were irradiated at 365 nm UV with 400 mJ/cm², then placed at 2-8°C to stand for 1, 5, 10, and 15 minutes, respectively, and blocking solution (1% human serum albumin/RPMI medium) was added.

### Step 4:

After the mixtures from Step 3 were centrifuged at 400g for 5 minutes at 4-8°C, the supernatant was removed, the cells were washed twice with 10 mL PBS, and finally the cells were resuspended in PBS and the cell concentration was adjusted to 5×10⁵ cells/mL for subsequent experiments.

### Step 5:

Lung cancer cells NCIH2170 were cultured in a 10 cm cell culture dish with 10 mL of cell culture medium (RPMI/10%FBS) at 37°C, 5% CO₂ for three days. The cell medium was removed and the cells were washed with 10 mL PBS, 1mL of CTS^{™} TrypLE^{™} Select enzyme was added, reacted at 37°C, 5% CO₂ for 5 minutes, and 10 mL of cell culture medium was added to collect the cells. After transferred to a 15 mL centrifuge tube and centrifuged at 400g for 5 minutes at 4-8°C, the supernatant was removed, and then the NCIH2170 cells were washed and centrifuged twice with PBS. Finally, the NCIH2170 cells were resuspended in PBS and the cell concentration was adjusted to 5×10⁶-1×10⁷ cells/mL.

### Step 6:

20 µL of DMSO solvent was added to a Deep Red reagent tube. 5 µL of Deep Red solution was taken and added to 5 mL of PBS to prepare a 1 µM CellTracke^{™} Deep Red dye (Invitrogen^{™}, C34565) solution, and stored and protected from light. 2 mL of breast cancer cells BT474 was mixed with 2 mL of the 1 µM Deep Red solution at a volume ratio of 1:1 to make a total of 4 mL, and reacted at room temperature, protected from light, for 10 minutes.

### Step 7:

10 mL of cell culture medium (RPMI/10%PBS) was taken to centrifuge and the Deep Red-stained NCIH2170 cells were washed twice at 400g for 5 minutes at 4-8°C. The cell concentration of Deep Red-stained BT474 breast cancer cells was adjusted to 1×10⁵ cells/mL using cell culture medium.

### Step 8:

The NK cells from the experimental group, control group, and comparison group in Step 4 were taken and mixed with the CellTracker^{™} Deep Red dye-stained BT474 cells at a ratio of 5:1 in a 96-well flat-bottom plate with a final volume of 200 µL, and reacted at 37°C for 5 hours.

### Step 9:

100 µL of Annexin V/PI solution (98.5 µL of 1X binding buffer plus 1 µL Annexin V and 0.5 µL PI) was prepared. 200 µL of cytotoxic effect cells after the 5 hours of reaction were taken and mixed with 100 µL of Annexin V/PI solution, reacted at room temperature, protected from light, for 5 minutes, and analyzed the Deep Red+/Annexin V⁺/PI⁺ cell population by flow cytometry (FACS).

### Step 10:

After 200 µL of solutions of each group from Step 4 was taken into 1.5 mL test tube (Eppendorf). Goat F(ab')2 Anti-Human IgG-(Fab')2 (PE) (abcam, cat.no. ab98606) was diluted in PBS at a volume ratio of 1:200, and 200 µL of this dilution was added to solutions of each group, and reacted at 4°C for 15-30 minutes. 1000µL PBS was added, after being centrifuged at 400g for 5 minutes at 4-8°C, the supernatant was removed, then the cells were washed and centrifuged twice with 1 mL PBS, the supernatant was removed, the cells were resuspended in 200µL PBS for each group, and analyzed by flow cytometry (FACS).

Finally, Goat F(ab')2 Anti-Human IgG-(Fab')2 (PE) and CD16 monoclonal antibody were used to carry out antibody staining on the NK cell membrane, followed by flow cytometry analysis of the PE signal to obtain the strength of Trastuzumab conjugating to NK cells.

### Experimental Results [Conjugation Rate Analysis]:

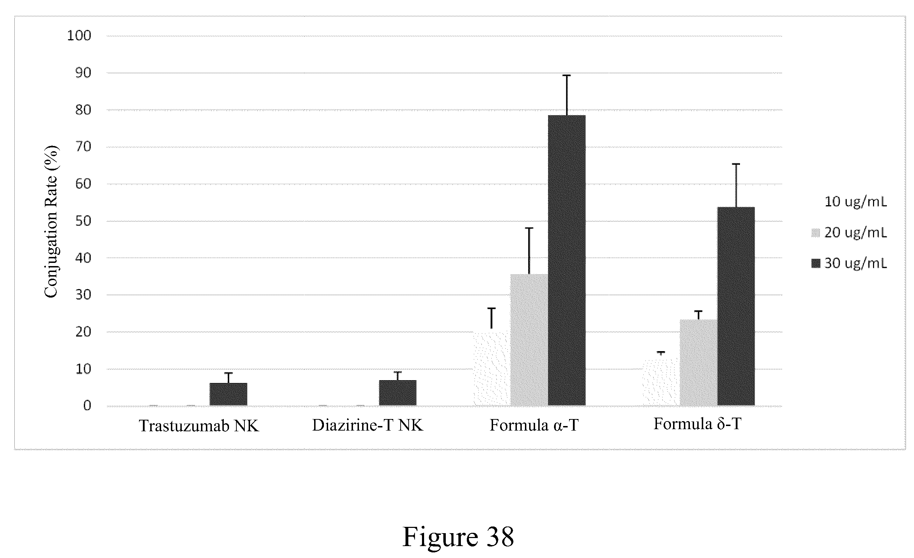

Figure 38 is a diagram that shows the analysis of the conjugating degree of different linker conjugated Trastuzumab to NK at different antibody concentrations. From Figure 38, it can be observed that different linker conjugated Trastuzumab reacted with NK at concentrations of 10, 20, and 30 µg/mL, and after irradiated with UV at 365 nm and reaction under low-temperature condition, compared to the Trastuzumab NK and the Diazirine-T NK cells in the commercially available photosensitive molecule comparison group, the formula α-T NK and formula δ-T NK were successfully conjugated to the surface of NK cells, in which formula α-T and formula δ-T exhibited higher conjugation ratios with NK cells at the concentration of 10 µg/mL.

### Experimental Results [Cytotoxicity Analysis]:

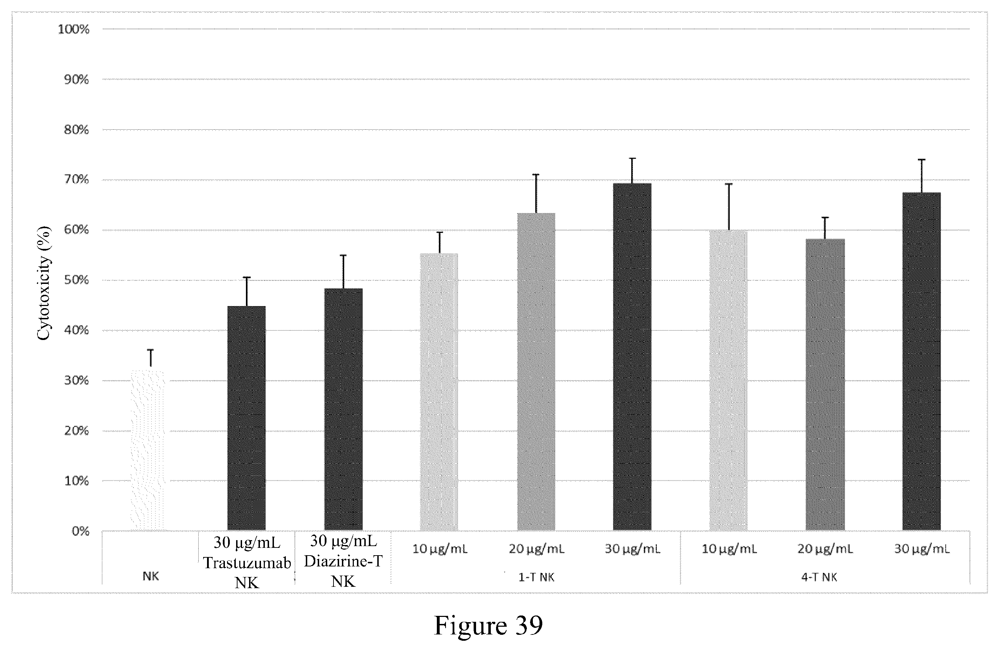

First, the Deep Red⁺ NCI-H2170 cell population was selected, then the number of dead cells (Annexin V and PI-stained cell) in the spontaneous cell death group in NCI-H2170 cells was subtracted from the number of dead cells in the control group/experimental group/comparison (Annexin V and PI-stained cell) group, then divided by the number of live NCI-H2170 cells ratio (Annexin V and PI-stained cell) for comparing the cytotoxic effects. Figure 39 is a diagram that shows the analysis of the cytotoxicity results of different hydrocarbon chain linker conjugated Trastuzumab after UV irradiation. From Figure 39, it can be observed that, compared to the NK cells in the control group (45±6%) and the Trastuzumab NK and Diazirine-T-NK in the comparison group (48±7%), the 1-T and 4-T NK cells in the experimental groups exhibited better cytotoxic effect, among them, formula 1-T NK (30µg/mL) cells had the best cytotoxic activity with cytotoxicity ratio of 69±5% at an E/T ratio of 5:1, and the cytotoxicity ratio increased along with the antibody concentration.

The foregoing outlines features of several embodiments so that those skilled in the art may better understand the aspects of the present invention. Those skilled in the art should appreciate that they may readily use the present invention as a basis for designing or modifying other processes and structures for carrying out the same purposes and/or achieving the same advantages of the embodiments introduced herein. Those skilled in the art should also realize that such equivalent constructions do not depart from the scope of the present invention, and that they may make various changes, substitutions, and alterations herein without departing from the scope of the present invention.

## Claims

1. A targeting molecule-cell complex having Formula (I):
X-A₁-L-A₂-D Formula (I)
wherein:
X is a cell;
A₁ is a substituted or unsubstituted indazolone moiety;
L is -O-(CH₂)ₘ-W-(CH₂)ₙ-, wherein m and n are each independently integers from 0 to 10, and W is a single bond, or -NHCO-, or a substituted or unsubstituted polyethylene glycol (PEG) with 1-4 units;
A₂ is -CONH- or -COS-; and
D is a targeting moiety.

2. The targeting molecule-cell complex according to claim 1, wherein A₁ is a methoxy-substituted indazolone group.

3. The targeting molecule-cell complex according to claim 2, having a structure of Formula (II) or (III): 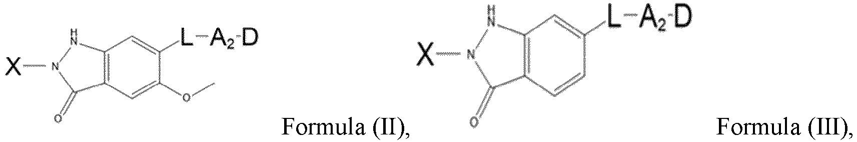 wherein X, L, A₂, and D are as defined in claim 1.

4. The targeting molecule-cell complex according to claim 1, wherein L is -O-(CH₂)ₘ₊ₙ-, and wherein 3 ≤ (m+n) ≤ 9.

5. The targeting molecule-cell complex according to claim 1, wherein L is -O-(CH₂)ₘ-NHCO-(CH₂)ₙ-, and 3≤(m+n)≤9.

6. The targeting molecule-cell complex according to claim 1, wherein L is the substituted or unsubstituted polyethylene glycol with 1-4 units.

7. The targeting molecule-cell complex according to claim 1, wherein the cell is a mesenchymal stem cell, a blood cell, or a bacterial cell.

8. The targeting molecule-cell complex according to claim 7, wherein the mesenchymal stem cell comprises adipose-derived mesenchymal stem cell (ADMSC), hematopoietic stem cell, bone marrow mesenchymal stem cell, umbilical cord mesenchymal stem cell, and embryonic stem cell.

9. The targeting molecule-cell complex according to claim 7, wherein the blood cell comprises a platelet, a T lymphocyte, a natural killer cell, a dendritic cell, a macrophage, a granule, or a combination thereof.

10. The targeting molecule-cell complex according to claim 1, wherein the targeting moiety is an antigen-binding molecule.

11. The targeting molecule-cell complex according to claim 10, wherein the targeting moiety comprises an amino group or a sulfhydryl group.

12. The targeting molecule-cell complex according to claim 10, wherein the targeting moiety is a small molecule, an aptamer, a peptide, an antibody, or a combination thereof.

13. The targeting molecule-cell complex according to claim 11, wherein the antibody is a single-chain variable fragment (scFv), a fragment antigen-binding (Fab) fragment, or a full-length antibody.

14. A method of preparing a targeting molecule-cell complex, comprising:
providing a linker having Formula (IV): 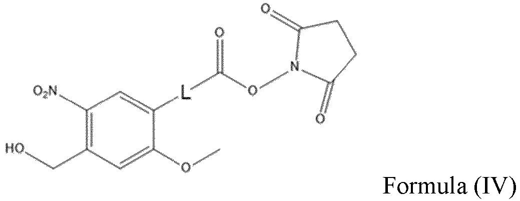
wherein L is -O-(CH₂)ₘ-W-(CH₂)ₙ-, wherein m and n are independently integers from 0 to 10, and W is a single bond, or -NHCO-, or a substituted or unsubstituted polyethylene glycol with 1-4 units;
reacting the linker with a targeting molecule containing an amino group to form a linker-targeting molecule complex, wherein the linker is bonded to the targeting molecule via an amide bond; and
irradiating the linker-targeting molecule complex with ultraviolet light, such that the irradiated linker-target molecule complex reacts with a cell, wherein the linker-targeting molecule complex reacts with the cell through an indazolone moiety to form a targeting molecule-cell complex.

15. The method of preparing a targeting molecule-cell complex according to claim 14, wherein the linker has a structure selected from the group consisting of the following compounds: 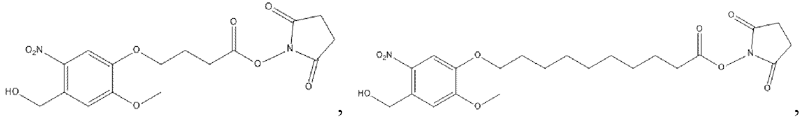 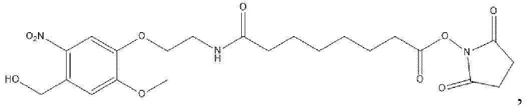 and

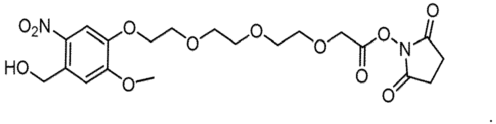

16. The method of preparing a targeting molecule-cell complex according to claim 14,
wherein a molar ratio of the targeting molecule to the linker used for forming the linker-targeting molecule complex is 1:1 to 1:15.

17. The method of preparing a targeting molecule-cell complex according to claim 14,
wherein the step of irradiating the linker-targeting molecule complex with ultraviolet light is carried out by irradiating the linker-target molecule complex for 5 to 30 seconds, followed by a standing period of 1 to 30 seconds in a cycle.

18. The method of preparing a targeting molecule-cell complex according to claim 14,
wherein the cycle is repeated 1 to 6 times.

19. The method of preparing a targeting molecule-cell complex according to claim 14,
wherein the step of reacting the irradiated linker-target molecule complex with the cell is carried out for at least 1 minute.

20. The method of preparing a targeting molecule-cell complex according to claim 14,
wherein the step of irradiating the linker-target molecule complex with ultraviolet light comprises first mixing the linker-target molecule complex with the cell, and then irradiating the linker-target molecule complex and the cell with ultraviolet light simultaneously.

21. Use of the targeting molecule-cell complex according to claim 1 in the preparation of a medicament for treating cancer.

22. Use of the targeting molecule-cell complex according to claim 1 in the preparation of a medicament for promoting cell homing.

23. Use of the targeting molecule-cell complex according to claim 1 in the preparation of a medicament for treating autoimmune diseases.
